# EUROPEAN PATENT SPECIFICATION

(11) **EP 2 836 271 B1**
(45) Date of publication and mention of the grant of the patent: **13.06.2018**
(21) Application number: 13775511.2
(22) Date of filing: 09.04.2013
(51) Int. Cl.: A61B 5/00, A61B 5/0488, A61B 5/11, A61N 1/05, A61N 1/36, A61N 1/372

(54) **IMPLANTABLE NEUROSTIMULATOR AND A PROGRAMMER FOR PROVIDING A MASSAGING SENSATION**
IMPLANTIERBARES STIMULATIONSGERÄT UND EIN PROGRAMMIERGERÄT ZUR ERZEUGUNG EINES MASSIERENDEN GEFÜHLS
NEUROSTIMULATEUR IMPLANTABLE ET UNE CONSOLE DE PROGRAMMATION POUR GÉNÉRER UNE SENSATION MASSANTE

(30) Priority: 09.04.2012 US 201261621948 P; 11.10.2012 US 201261712731 P; 15.03.2013 US 201313839324
(43) Date of publication of application: 18.02.2015
(73) Proprietor: Spinal Modulation, Inc., Menlo Park, CA 94025 (US)
(72) Inventor: NIJHUIS, Harold, 3707 ZH Zeist (NL); WOOD, David S., Mountain View, CA 94041 (US); BROUNSTEIN, Daniel, M., San Francisco, CA 94110 (US); KRAMER, Jeffery, M., San Francisco, CA 94123 (US); AIKEMA, Gerry, M., 5045 CA Tilburg (NL)
(74) Representative: Booth, Catherine Louise
(86) International application number: PCT/US2013/035853
(87) International publication number: WO 2013/155117

(56) References cited:
- WO-A1-2006/091611
- WO-A2-2008/070807
- WO-A2-2010/062622
- US-A1- 2006 116 737
- US-A1- 2009 005 833
- US-A1- 2010 125 304
- US-A1- 2010 274 327
- US-A1- 2011 004 269
- US-A1- 2012 022 615

## Description

### STATEMENT AS TO RIGHTS TO INVENTIONS MADE UNDER FEDERALLY SPONSORED RESEARCH AND DEVELOPMENT

NOT APPLICABLE

### REFERENCE TO A "SEQUENCE LISTING," A TABLE, OR A COMPUTER PROGRAM LISTING APPENDIX SUBMITTED ON A COMPACT DISK.

NOT APPLICABLE

### BACKGROUND OF THE INVENTION

Pain of any type is the most common reason for physician consultation in the United States, prompting half of all Americans to seek medical care annually. It is a major symptom in many medical conditions, significantly interfering with a person's quality of life and general functioning. Diagnosis is based on characterizing pain in various ways, according to duration, intensity, type (dull, burning, throbbing or stabbing), source or location in body. Usually if pain stops without treatment or responds to simple measures such as resting or taking an analgesic, it is then called acute pain. But it may also become intractable and develop into a condition called chronic pain in which pain is no longer considered a symptom but an illness by itself.

It has been reported that more than 1.5 billion people worldwide suffer from chronic pain and that approximately 3- 4.5% of the global population suffers from neuropathic pain, pain resulting from damage or disease affecting the somatosensory system. Chronic pain can be mild or excruciating, episodic or continuous, merely inconvenient or totally incapacitating.

Of suffers of chronic pain, low back pain is particularly prevalent and causes significant debilitation. When asked about four common types of pain, respondents of a National Institute of Health Statistics survey indicated that low back pain was the most common (27%), followed by severe headache or migraine pain (15%), neck pain (15%) and facial ache or pain (4%). Back pain is the leading cause of disability in Americans under 45 years old. More than 26 million Americans between the ages of 20-64 experience frequent back pain. Adults with low back pain are often in worse physical and mental health than people who do not have low back pain: 28% of adults with low back pain report limited activity due to a chronic condition, as compared to 10% of adults who do not have low back pain. Also, adults reporting low back pain were three times as likely to be in fair or poor health and more than four times as likely to experience serious psychological distress as people without low back pain.

A significant portion of patients suffering from low back pain are experiencing referred pain due to the Thoracolumbar Junction Syndrome (also known as TLJ Syndrome, Maigne syndrome, posterior ramus syndrome and dorsal ramus syndrome). Thoracolumbar Junction Syndrome is defined by a dysfunction of the thoracolumbar junction (TLJ) referring pain in the whole or part of the territory of the corresponding dermatomes (eg. T11 to L1 or L2). Depending on the branch involved, the pain could refer to the low back (cutaneous dorsal rami), to the groin (subcostal or iliohypogastric nerve) or in the lateral aspect of the hip (lateral cutaneous rami of the subcostal or iliohypogastric nerve or cluneal nerve). All combinations of these clinical presentations are possible with one, two or three involved territories.

Low back pain is the most frequently encountered pain complaint in the TLJ syndrome. The pain is distributed in the dermatomes of T11, T12, L1 or L2. Because the limits of these dermatomes are ill defined, due to overlapping and anastomosis, the pain is usually spread in the lateral part of the low back without corresponding exactly to a specific dermatome. Rarely, the pain is bilateral; more often, it is unilateral. The pain is usually acute, of less than 2 or 3 months duration, often appearing after a false rotatory movement of the trunk, prolonged strenuous posture, lifting and occasionally, without any obvious precipitating factors.

Since the dermatomes covering the groin are T12, L1 and L2, groin pain is easily related to a TLJ origin. Groin pain may accompany low back pain or be an isolated complaint. The pain may sometimes be located above the groin, in the T10 or T11 dermatomes, depending on the involved level of the TLJ.

The third feature of the TLJ syndrome is pain over the lateral aspect of the hip. It is a referred pain in the territory of the lateral cutaneous branch of either the iliohypogastric or the subcostal nerve or an extension of the cluneal nerve.

Each of the different pain syndromes characterizing the TLJ syndrome can appear in isolation or in combination in a given patient. The most common cause is a dysfunction at T10/T11, T11/T12 or T12/L1. This area is vulnerable as a large percentage of the total rotation in the spine comes from the TLJ. As such when other segments become restricted the TLJ may be over utilised in rotation either acutely or chronically resulting in dysfunction within the motion segment. The nature of this dysfunction remains unknown, although the involvement of either the facets or the disc is very likely. Some other causes are possible, although very rare, such as a disc herniation or a collapse of the vertebral body of T11, L2 or L1 referring pain only in the low back.

The treatment of the TLJ syndrome is at first the treatment of the TLJ itself. The TLJ syndrome can be responsive to spinal manipulative therapy. Manipulation is a forced movement applied to a joint within the anatomic limits. This movement is characterized by a cracking sound due to a vacuum phenomenon as the facets separate. The vacuum phenomenon, or cavitation, makes the separation of the articular surfaces very sudden, even more so than the movement which initiated it. Thus, the cavitation appears as a motion accelerator, which could play a role by stretching hypertonic muscles. This is true not only for the TLJ but also for any part of the spine. The separation of the facets could also unblock the motion segment. Manipulation may also act on the disc. This could alter the load transmission through the disc, thought to be one of the factors transforming a pain free degenerated disc into a painful one.

If manipulative treatment is unsuccessful or contraindicated for a particular patient, such as in an elderly patient where osteoarthritis is likely, facet injections can be performed. A facet injection is an injection of a steroid in the facet joints which are located in the back area the spinal bony structure. The steroid injected reduces the inflammation and/or swelling of tissue in the joint space. This may in turn reduce pain and other symptoms caused by inflammation or irritation of the joint and surrounding structures. The effects of facet injections tend to be temporary, providing relief for several days or three to six months.

If facet injections are successful, longer relief may be provided by a facet rhizotomy, a procedure that uses an electrical current to destroy the nerve fibers carrying pain signals to the brain. Using a local anesthetic and x-ray guidance, a needle with an electrode at the tip is placed alongside the small nerves to the facet joint. The electrode is then heated, with a technology called radio frequency, to deaden these nerves that carry pain signals to the brain. The effects of facet rhizotomy are also temporary, providing relief for two months to 1 year.

Improved treatments are desired to provide more lasting relief to patients suffering from both TLJ syndrome and other ailments causing chronic pain, particularly low back pain. Likewise, improved treatments are desired to treat patients who are poor candidates for existing treatments or have not received adequate pain relief.

For other types of chronic pain, nerve stimulation has been utilized. For example, the application of specific electrical energy to the spinal cord has been actively practiced since the 1960s for the purpose of managing pain. It is known that application of an electrical field to spinal nervous tissue can effectively mask certain types of pain transmitted from regions of the body associated with the stimulated nervous tissue. Such masking is known as paresthesia, a subjective sensation of numbness or tingling in the afflicted bodily regions. Such electrical stimulation of the spinal cord, once known as dorsal column stimulation, is now referred to as spinal cord stimulation or SCS.

Alternatively, in some cases, peripheral neurostimulation or a combination of spinal and peripheral stimulation is applied. In the case of peripheral stimulation, one or more electrodes are subcutaneously placed in the area where the pain is localized and are subcutaneously connected with an implantable generator. The electric energy produced acts on the peripheral nerves in order to reduce pain. The document US 2012/022615 A1 describes an implantable neurostimulator and an associated programmer.

For evaluating the therapeutic result of nerve stimulation, a testing period of a few days takes place. Typically, pain relief of at least 50% and consent is required before a permanent system is implanted. The programming of the system is done telemetrically with a remote control. Programming selects and applies various stimulation parameter combinations that provide the optimal therapeutic result. The patient also is equipped with a remote control to turn off, activate and turn up and down the stimulator. In the case that there is no satisfactory result, the electrode(s) placed are withdrawn and removed. It is a fully reversible therapy, meaning that every neuromodulation system can at any time be removed in case of necessity.

Although conventional SCS and peripheral stimulation systems have effectively relieved pain in some patients, these systems have a number of drawbacks. To begin, in conventional SCS, the lead is positioned upon the spinal cord dura layer near the midline of the spinal cord. The electrodes stimulate a wide portion of the spinal cord and associated spinal nervous tissue. Significant energy is utilized to penetrate the dura layer and cerebral spinal fluid to activate fibers in the spinal column extending within the posterior side of the spinal cord. Sensory spinal nervous tissue, or nervous tissue from the dorsal nerve roots, transmit pain signals. Therefore, such stimulation is intended to block the transmission of pain signals to the brain with the production of a tingling sensation (paresthesia) that masks the patient's sensation of pain. However, excessive tingling may be considered undesirable. Further, the energy also typically penetrates the anterior side of the spinal cord, stimulating the ventral horns, and consequently the ventral roots extending within the anterior side of the spinal cord. Motor spinal nervous tissue, or nervous tissue from ventral nerve roots, transmits muscle/motor control signals. Therefore, electrical stimulation by the lead often causes undesirable stimulation of the motor nerves in addition to the sensory spinal nervous tissue. The result is undesirable muscle contraction.

Because the electrodes span several levels and because they stimulate medial to spinal root entry points, the generated stimulation energy stimulates or is applied to more than one type of nerve tissue on more than one level. Moreover, these and other conventional, non-specific stimulation systems also apply stimulation energy to the spinal cord and to other neural tissue beyond the intended stimulation targets. As used herein, non-specific stimulation refers to the fact that the stimulation energy is provided to multiple spinal levels including the nerves and the spinal cord generally and indiscriminately. This is the case even with the use of programmable electrode configurations wherein only a subset of the electrodes are used for stimulation. In fact, even if the epidural electrode is reduced in size to simply stimulate only one level, that electrode will apply stimulation energy non-specifically and indiscriminately (i.e. to many or all nerve fibers and other tissues) within the range of the applied energy.

One of the drawbacks of conventional peripheral stimulation is the large lead migration rates. Often soft tissue anchors are inadequate in keeping the lead in the desired position, primarily due to the movement of tissues in the periphery which can place mechanical forces on the lead instigating migration. This has been observed in several locations within the body from the lower extremities, to the occipital region as well as areas in the axial trunk.

Therefore, an improved stimulation system, is desired that enables more precise and effective delivery of stimulation energy. In addition, an improved stimulation system, is desired that provides treatment with minimal undesired side effects and loss of efficacy over time, such as due to migration or device failure.

### SUMMARY OF THE INVENTION

Aspects of the present disclosure provide a system for modulating portions of the nervous system.

In a first aspect, a method is provided of treating pain in a patient. In some cases, the method comprises positioning a lead having at least one electrode so that at least one of the at least one electrodes is near a dorsal root ganglion associated with the pain, and selecting at least one stimulation parameter so that energy is provided to the at least one of the at least one electrodes which results in selective stimulation of at least a portion of the dorsal root ganglion, wherein the selective stimulation causes a massaging sensation which treats the pain.

In some cases, positioning the lead further comprises positioning the lead so that at least one of the at least one electrodes is near a mixed spinal nerve, a dorsal ramus a lateral branch, a medial branch, an intermediate branch and/or a ventral ramus associated with the dorsal root ganglion. In such cases, selecting at least one stimulation parameter may further comprise selecting at least one stimulation parameter so that energy is provided to at least one of the electrodes which results in selective stimulation of the mixed spinal nerve, the dorsal ramus, a lateral branch, a medial branch, an intermediate branch and/or the ventral ramus.

In some cases, the dorsal root ganglion is disposed on spinal level T10, T11, T12, L1, L2, L3, L4 or L5. In particular, in some embodiments, the dorsal root ganglion is disposed on spinal level L2 or L3. Likewise, in some cases, the pain is associated with a thoracolumbar junction of the patient. Similarly, the pain may be located in a low back of the patient.

In some cases, the method further comprises selecting at least one stimulation parameter so that the selective stimulation causes undesired muscle contraction prior to selecting the at least one stimulation parameter so that the selective stimulation causes the massaging sensation.

In some cases, selecting at least one stimulation parameter comprises selecting a frequency of at least 16 Hz. For example, in some cases, selecting at least one stimulation parameter comprises selecting a frequency in the range of approximately 20-50 Hz. Or, in other cases, selecting at least one stimulation parameter comprises selecting a frequency in the range of approximately 20 Hz. In some cases, the method further comprises selecting a frequency of less than or equal to 10 Hz so that a motor contraction is generated and then increasing the frequency to the frequency of at least 16 Hz to cause the massaging sensation.

In another aspect a method is provided of treating a condition of a patient comprising advancing a lead having at least one electrode into an epidural space in an antegrade direction, laterally away from a midline of a spinal cord and through a foramen so that at least one of the at least one electrodes is positioned near a peripheral nerve associated with the condition, and selecting at least one stimulation parameter so that energy is provided to the at least one of the at least one electrodes which results in selective stimulation of at least a portion of the peripheral nerve, wherein the selective stimulation treats the condition.

In some cases, the selective stimulation causes a massaging sensation. In some cases, the peripheral nerve comprises a mixed spinal nerve. For example, in some cases, the peripheral nerve comprises a dorsal ramus. For example, in some cases, the peripheral nerve comprises a lateral branch, a medial branch or an intermediate branch. In other cases, the peripheral nerve comprises a ventral ramus.

In some cases, the method further comprises selecting at least one stimulation parameter so that energy is provided to at least one electrode which results in selective stimulation of at least a portion of a dorsal root ganglion disposed within the foramen.

In some cases, the foramen is disposed on spinal level T10, T11, T12, L1, L2, L3, L4 or L5. In particular, the foramen may be disposed on spinal level L2 or L3. In some cases, the condition comprises pain associated with a thoracolumbar junction of the patient. In some cases, the condition comprises pain located in a low back of the patient.

In some cases, selecting at least one stimulation parameter comprises selecting a frequency of at least 16 Hz. In particular, in some cases, selecting at least one stimulation parameter comprises selecting a frequency in the range of approximately 20-50 Hz. Likewise, in some cases, selecting at least one stimulation parameter comprises selecting a frequency of approximately 20 Hz.

In some cases, the method further comprises selecting a frequency of less than or equal to 10 Hz so that a motor contraction is generated and then increasing the frequency to the frequency of at least 16 Hz to treats the condition by generating a massaging sensation.

Some cases relate to a system configured for treating pain in a patient. Such a system includes an implantable neurostimulator (also referred to as an implantable pulse generator (IPG)), an implantable lead and a non-implantable programmer. The implantable neurostimulator includes a controller, memory and telemetry circuitry. The implantable lead, which is connected to the neurostimulator, has least one electrode configured to be positioned near a dorsal root ganglion associated with the pain. The non-implantable programmer, which includes a controller, memory and telemetry circuitry, is configured to select at least one stimulation parameter used to provide energy to the at least one of the at least one electrodes which results in selective stimulation of at least a portion of the dorsal root ganglion, wherein the selective stimulation causes a massaging sensation which treats the pain. The telemetry circuitry of the programmer is configured to send instructions to the neurostimulator to cause the energy to be provided to the at least one of the at least one electrodes and thereby cause the massaging sensation which treats the pain.

In some cases, the lead further comprises at least one further electrode, wherein at least one of the at least one further electrodes is configured to be positioned near a mixed spinal nerve, a dorsal ramus , a lateral branch, a medial branch, an intermediate branch and/or a ventral ramus associated with the dorsal root ganglion while the at least one of the at least one electrodes is positioned near the dorsal root ganglion. In somme cases, the programmer is also configured to select at least one stimulation parameter used to provide energy to the at least one of the at least one further electrode which results in selective stimulation of the mixed spinal nerve, the dorsal ramus, the lateral branch, the medial branch, the intermediate branch and/or the ventral ramus. In some cases, the at least one stimulation parameter comprises selection of the at least one of the at least one electrode and/or the at least one of the further electrode to receive stimulation. In some cases, the at least one stimulation parameter comprises frequency, amplitude or pulse width. In specific cases, the at least one stimulation parameter comprises a frequency of at least 16 Hz. In specific cases, the at least one stimulation parameter comprises a frequency in the range of approximately 20-50 Hz. In specific cases, the at least one stimulation parameter comprises a frequency of approximately 20 Hz.

In some cases, the programmer is further configured to select at least one stimulation parameter used to provide energy to the at least one of the at least one electrodes which results in an undesired muscle contraction, wherein the undesired muscle contraction is indicative of the at least one of the at least one electrodes being positioned near the dorsal root ganglion associated with the pain. The at least one stimulation parameter used to provide energy to the at least one of the at least one electrodes which results in undesired muscle contractions has a frequency of less than or equal to approximately 10 Hz. The system includes a sensor configured to detect the resultant undesired muscle contraction and the programmer is configured to enable an increase in the frequency to at least 16Hz to transition the undesired muscle contractions to the massaging sensation which treats the pain.

In somme cases, the programmer is configured to cause testing, by the implantable neurostimulator, of a plurality of different combinations of electrodes to identify which combination of the electrodes results in undesired muscle contractions when used to selectively stimulate at least a portion of the dorsal root ganglion at a first frequency. The programmer is configured to increase the first frequency to a second frequency to transition the undesired muscle contractions to the massaging sensation which treats the pain, after the combination of the electrodes which results in the undesired muscle contractions is identified. The first frequency is less than or equal to approximately 10Hz and the second frequency is at least 16Hz. Furthermore, a sensor is configured to detect the undesired muscle contractions.

In some cases, the lead further comprises at least one further one of the at least one electrodes configured to be positioned near a mixed spinal nerve, a dorsal ramus, a lateral branch, a medial branch, an intermediate branch and/or a ventral ramus associated with the dorsal root ganglion. In some cases, the programmer is also configured to select at least one stimulation parameter used to provide energy to the at least one further one of the at least one electrodes which results in selective stimulation of the mixed spinal nerve, the dorsal ramus, a lateral branch, a medial branch, an intermediate branch and/or the ventral ramus.

The system also includes a sensor configured to detect when selective stimulation, having a frequency of less than or equal to 10Hz, causes undesired muscle contractions, which are indicative of the at least one of the at least one electrodes being positioned near the dorsal root ganglion associated with the pain. Furthermore, the programmer is configured to enable an increase in the frequency to at least 16Hz to transition the undesired muscle contractions to the massaging sensation which treats the pain.

Some cases relate to a computer readable medium, including instructions stored thereon which when read and executed by one or more processors cause the one or more processors to perform certain steps. Such steps may include causing testing of a plurality of different stimulation parameters that are used for selective stimulation of at least a portion of a dorsal root ganglion associated with pain in a patient that is to be treated. Such steps can also include identifying when the selective stimulation causes a massaging sensation which treats the pain, as well as saving information corresponding to at least one stimulation parameter that causes the massaging sensation which treats the pain. In one case, the identifying includes receiving an indication that the selective stimulation causes a massaging sensation which treats the pain. In one case, the steps include identifying when selective stimulation causes an undesired muscle contraction indicative of at least one electrode being used to deliver the selective stimulation being positioned near the dorsal root ganglion associated with the pain. In a certain case, the identifying when selective stimulation causes an undesired muscle contraction comprises sensing when selective stimulation causes an undesired muscle contraction. The identifying further comprises increasing a frequency of less than or equal to 10 Hz to a frequency of least 16Hz to transition the undesired muscle contraction to the massaging sensation which treats the pain.

Objects and advantages of the present invention will become apparent from the detailed description to follow, together with the accompanying drawings.

### BRIEF DESCRIPTION OF THE DRAWINGS

Fig. 1A illustrates a stimulation system.
Fig. 1B illustrates additional details of the IPG of Fig. 1A.
Fig. 1C illustrates additional details of the programmer of Fig. 1A.
Fig. 2 illustrates example placement of the leads of the system of Fig. 1 within the patient anatomy.
Fig. 3 illustrates a cross-sectional view of an individual spinal level showing a lead of the stimulation system positioned near a target dorsal root ganglion.
Fig. 4 illustrates a pair of spinal nerves extending from the spinal cord at a spinal level.
Fig. 5 illustrates an example distribution of spinal nerve branches within a patient.
Figs. 6A-6C illustrate example areas of referred pain.
Fig. 7 illustrates spinal nerve branches extending from the vertebral column, including a lead positioned so as to selectively stimulate a dorsal root ganglion.
Fig. 8 illustrates a case of optional lead placement wherein the distal end of the lead is extended through the foramen so as to position at least one electrode near the mixed nerve.
Fig. 9 illustrates a case wherein the lead is advanced so that at least one electrode is positioned along the medial branch of the dorsal ramus.
Fig. 10 illustrates a case wherein the lead is advanced further through the foramen so that at least one electrode is positioned along a portion of the ventral ramus.
Figs. 11A-11B illustrate a lumbar plexus and a lead positioned so as to selectively stimulate a portion of the lumbar plexus.
Fig. 12 illustrates a lead positioned so as to selectively stimulate the trigeminal ganglion.
Fig. 13 illustrates a lead positioned so as to selectively stimulate a portion of a cervical plexus.
Figs. 14, 15A-15B illustrate a brachial plexus and a lead positioned so as to selectively stimulate a portion of the brachial plexus.
Fig. 16A illustrates a cross-sectional view of the thoracic cavity and associated spinal anatomy.
Fig. 16B illustrates a lead positioned so as to selectively stimulate a portion of an intercostal nerve.
Figs. 17A-17B illustrate a sacral plexus and a lead positioned so as to selectively stimulate a portion of the sacral plexus.
Figs. 18A-18D illustrate a lead and delivery system.
Fig. 19 illustrates a sheath advanced over the shaft of a lead until a portion of its distal end abuts the distal tip of the lead.
Fig. 20 illustrates a stylet disposed within a lead so that extension of the lead and stylet through the sheath bends or directs the lead.
Fig. 21 illustrates an assembled sheath/lead/stylet that is advanced within the epidural space toward a DRG.
Fig. 22 illustrates the lead/stylet of Fig. 21 able to be advanced beyond the distal end of the sheath along a nerve root.

### DETAILED DESCRIPTION OF THE INVENTION

The present system provides for targeted treatment of a variety of conditions with minimal deleterious side effects, such as undesired stimulation of unaffected body regions. This is achieved by directly neuromodulating a target anatomy associated with the condition while minimizing or excluding undesired neuromodulation of other anatomies. In most cases, neuromodulation comprises stimulation, however it may be appreciated that neuromodulation may include a variety of forms of altering or modulating nerve activity by delivering electrical or pharmaceutical agents directly to a target area. Descriptions herein will be provided in terms of stimulation and stimulation parameters.

In some cases, the systems and devices are used to stimulate portions of neural tissue of the central nervous system, wherein the central nervous system includes the spinal cord and the pairs of nerves along the spinal cord which are known as spinal nerves. The spinal nerves include both dorsal and ventral roots which fuse to create a mixed nerve which is part of the peripheral nervous system. At least one dorsal root ganglion (DRG) is disposed along each dorsal root prior to the point of mixing. Thus, the neural tissue of the central nervous system is considered to include the dorsal root ganglions and exclude the portion of the nervous system beyond the dorsal root ganglions, such as the mixed nerves of the peripheral nervous system. In some cases, the systems and devices are used to stimulate one or more dorsal root ganglia, dorsal roots, dorsal root entry zones, or portions thereof, while minimizing or excluding undesired stimulation of other tissues, such as surrounding or nearby tissues, ventral root and portions of the anatomy associated with body regions which are not targeted for treatment. In other cases, the systems and devices are used to stimulate portions of the peripheral nervous system. And, in still other cases, the systems and devices are used to stimulate other tissues including those described and illustrated herein. In some cases, example methods and devices for selectively stimulating target tissues are provided in US Patent Nos. 7,502,651; 7,337,005; 7,337,006; 7,450,993; 7,580,753; 7,447,546.

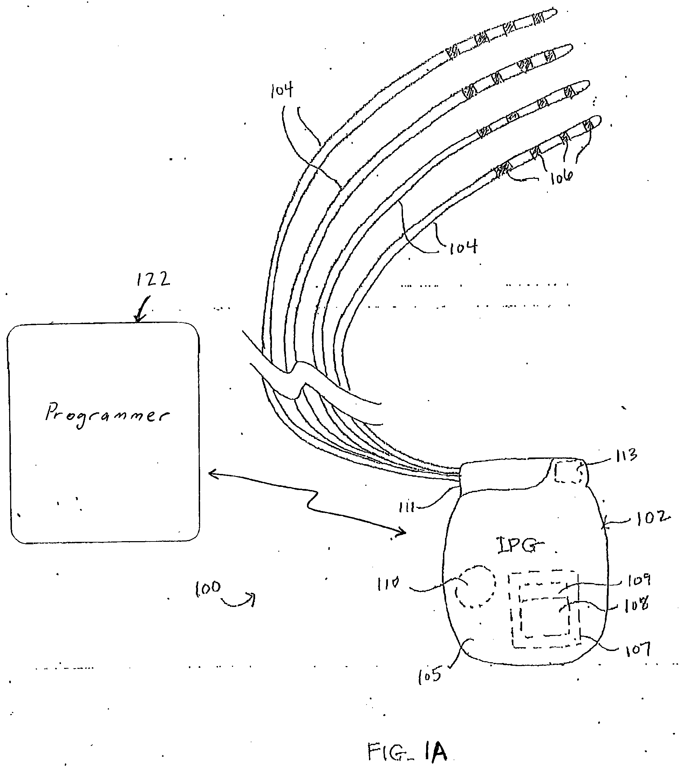

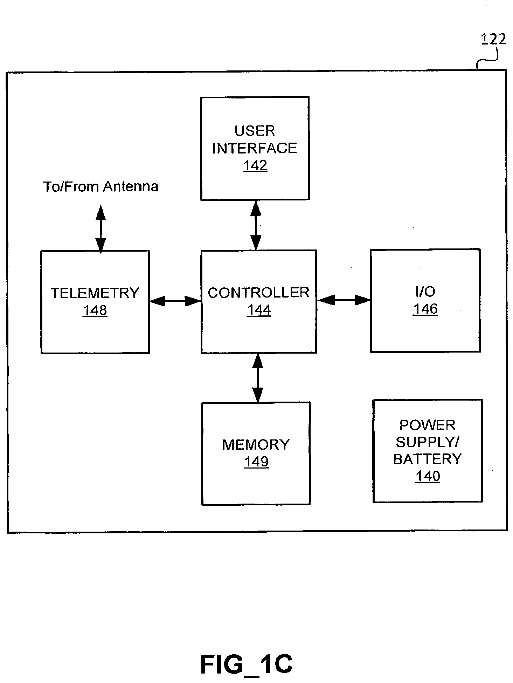

Fig. 1A illustrates a stimulation system 100 for treatment of a patient. The system 100 includes an implantable pulse generator (IPG) 102 and at least one lead 104 connectable thereto. In preferred cases, the system 100 includes four leads 104, as shown, however any number of leads 104 may be used including one, two, three, four, five, six, seven, eight, up to 58 or more. Each lead 104 includes at least one electrode 106. In preferred cases, each lead 104 includes four electrodes 106, as shown, however any number of electrodes 106 may be used including one, two, three, four five, six, seven, eight, nine, ten, eleven, twelve, thirteen, fourteen, fifteen, sixteen or more. Each electrode can be configured as off, anode or cathode. In some cases, even though each lead and electrode are independently configurable, at any given time the software ensures only one lead is stimulating at any time. In other cases, more than one lead is stimulating at any time, or stimulation by the leads is staggered or overlapping. The system 100 is also shown as including a non-implantable programmer 122 that is configured to communicate with the IPG 102. The IPG 102 can also be referred to as an implantable neurostimulator 102. Additional details of the programmer 122 are discussed below with reference to Fig. 1C.

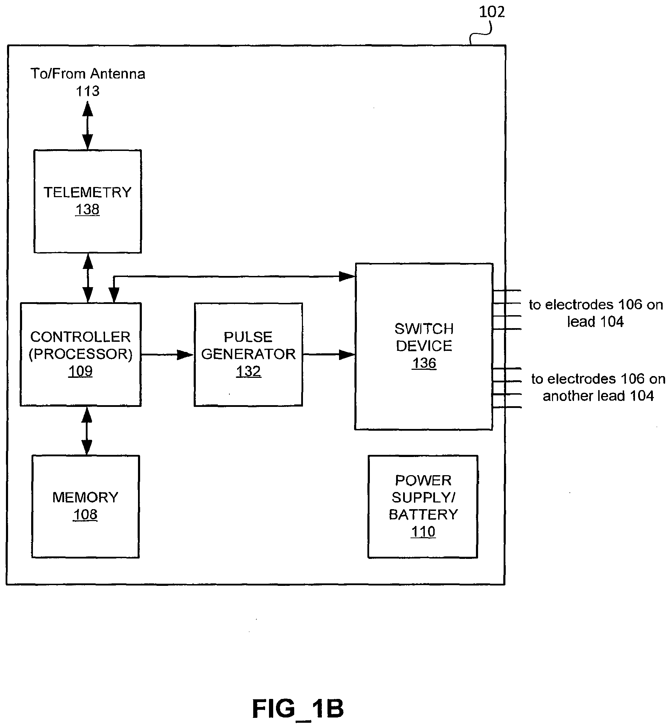

Referring again to Fig. 1A, the IPG 102 includes electronic circuitry 107 as well as an antenna 113 and power supply 110, e.g., a battery, such as a rechargeable or non-rechargeable battery, so that once programmed and turned on, the IPG 102 can operate independently of external hardware. In some cases, the electronic circuitry 107 includes a processor 109 and programmable stimulation information in memory 108. The electronic circuitry 107, memory 108, processor 109 and power supply 110 are located within a hermetically sealed housing 105. Fig. 1B is a simplified block diagram that is used to provide additional details of the IPG 102.

Referring to Fig. 1B, the IPG 102 is shown as including the processor 109 (or more generally, a controller 109), the power supply 110, the memory 108, a pulse generator 132, a switch device 136 and telemetry circuitry 138. The power supply 110 can include a battery that is used to power the various other components of the IPG 102. Further, the power supply 110 can be used to generate stimulation pulses. As such, the power supply can be coupled to the pulse generator 132, the controller 109, the switch device 136, the telemetry circuitry 138 and the memory 108. One or more voltage regulators (not shown) of the power supply 110 can step up or step down a voltage provide by a battery to produce one or more predetermined voltages useful for powering such components of the IPG 102.

The pulse generator 132 can be coupled to the electrodes 106 of the lead(s) 104 via the switch device 136. The pulse generator 132 can include circuitry, such as capacitors, resistors and transistors, which are used to generate stimulation pulses, as is well known in the art. The pulse generator 132 can be a single- or multi-channel pulse generator, and can be capable of delivering a single stimulation pulse or multiple stimulation pulses at a given time via a single electrode combination or multiple stimulation pulses at a given time via multiple electrode combinations.

The controller 109 can control the pulse generator 132 to generate stimulation pulses, and control the switch device 136 to couple the stimulation energy to selected electrodes 106 of one or more selected leads 104. Additionally, the controller 109 can control the switch device 136 to select different electrode configurations for delivery of stimulation energy from the pulse generator 132. For example, the controller 109 can control the pulse generator 132 and the switch device 136 to deliver stimulation energy in accordance with parameters specified by one or more sets of stimulation parameters, which may be stored within the memory 108. Each set of stimulation parameters can specify a lead, an electrode configuration for the specified lead, and one or more pulse parameters. For example, where there are four leads, a set of stimulation parameters can specify which of the four leads is selected. Where each lead includes four electrodes, a set of stimulation parameters can specify how each of the four electrodes of a selected lead is configured, e.g., as an anode (having a positive polarity), a cathode (having a negative polarity) or as an inactive electrode (in which case the electrode is not used for delivering stimulation energy). Exemplary programmable pulse parameters that can be specified include pulse amplitude, pulse width and pulse frequency (also known as pulse repetition rate) for a stimulation signal.

The controller 109 can include a microprocessor, a microcontroller, a digital signal processor (DSP), an application specific integrated circuit (ASIC), a field-programmable gate array (FPGA), a state machine or similar discrete and/or integrated logic circuitry. The switch device 136 can include a switch array, switch matrix, multiplexer, and/or any other type of switching device suitable to selectively couple stimulation energy to selected electrodes. The memory 108 can include RAM, ROM, NVRAM, EEPROM or flash memory. Various stimulation parameters can be stored in the memory 108, examples of which are discussed herein.

Fig. 1C is a simplified block diagram that illustrates possible components of the programmer 122 shown in Fig. 1A, wherein the programmer 122 can be a clinical programmer or a patent programmer. Referring to Fig. 1C, the programmer 122 is shown as including a power supply 140, a user interface 142, a controller 144, input and output (I/O) circuitry 146, telemetry circuitry 148 and a memory 149.

The power supply 140, which can include a battery, can be used to power the various other components of the programmer 122. As such, the power supply 140 can be coupled to the user interface 142, the controller 144, the I/O circuitry 146, the telemetry circuitry 148 and the memory 149. One or more voltage regulators (not shown) of the power supply 140 can step up or step down a voltage provided by a battery or an external power source to produce one or more predetermined voltages useful for powering such components of the programmer 122.

A programming person may interact with the controller 144 via the user interface 142 in order to test various stimulation parameters, input user feedback, select preferred or optimal stimulation parameters. The user interface 142 can include a display, a keypad, a touch screen, mechanical buttons, one or more peripheral pointing devices (e.g., a mouse, touchpad, joystick and/or trackball). The controller 144 can provide a graphical user interface (GUI) via the user interface 142 to facilitate interaction with a clinician or physician. Alternative types of user interfaces, e.g., one that relies primarily on mechanical type button, knows, switches may also be used. It is also possible that the programmer 122 include voice recognition capabilities, so that feedback received from the patient and/or programming person can be accepted verbally by the programmer 122. The controller 144 can include a microprocessor, a microcontroller, a digital signal processor (DSP), an application specific integrated circuit (ASIC), a field-programmable gate array (FPGA), a state machine, or similar discrete and/or integrated logic circuitry. The I/O circuitry 146 can include one or more transceivers for wireless communication, ports for wired communication and/or communication via removable electrical media and/or appropriate drives for communication via removable magnetic or optical media. The telemetry circuitry 148 can include, e.g., an RF transceiver that is connected to an antenna.

The controller 144 can collect information relating to tested stimulation parameters, and store the information in the memory 149 for later retrieval and review by a clinician, physician or by the controller 144 to facilitate identification of one or more preferred stimulation parameters, or sets thereof. The controller 144 can send instructions to the IPG 102 via the telemetry circuit 148 to cause the testing and/or selecting of various stimulation parameters. For example, the controller 144 can effectuate testing, by the IPG 102, of various sets of stimulation parameters created by the controller 144 or a programming person.

The memory 149 can include program instructions that, when executed by the controller 144, cause the programmer 122 to perform at least some of the functions described herein. For example, the controller 144 can execute program instructions that specify protocols for testing various sets of stimulation parameters and selecting one or more preferred sets of stimulation parameters. The memory 149 can also store one or more sets of stimulation parameters determined to treat targeted pain for a patient, along with information about the patient. The memory 149 can include any volatile, non-volatile, fixed, removable, magnetic, optical, or electrical media, such as a RAM, ROM, CD-ROM, hard disk, removable magnetic disk, memory cards or sticks, NVRAM, EEPROM and flash memory.

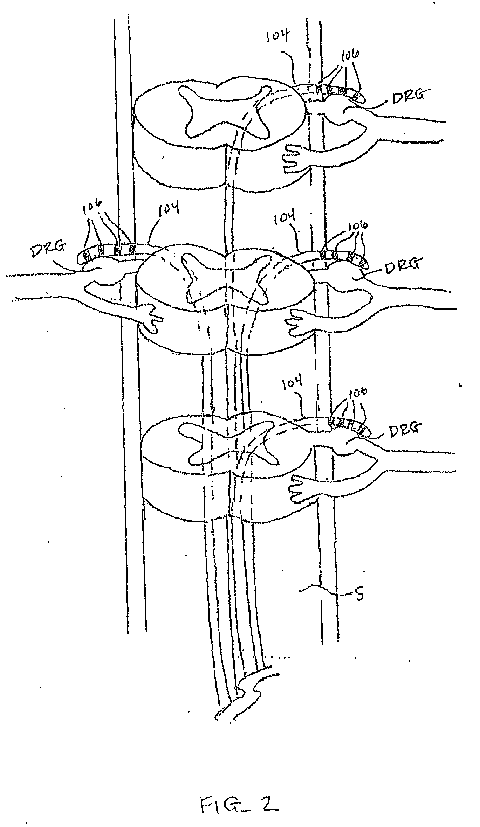

The implantable stimulation system 100 can be used to stimulate a variety of anatomical locations within a patient's body. In some cases, the system 100 is used to stimulate one or more dorsal roots, particularly one or more dorsal root ganglions. Fig. 2 illustrates example placement of the leads 104 of the system of Fig. 1 within the patient anatomy. In this example, each lead 104 is individually advanced within the spinal column S in an antegrade direction. Each lead 104 has a distal end which is guidable toward a target DRG and positionable so that its electrodes 106 are in proximity to the target DRG. In particular, Fig. 2 illustrates the stimulation of four DRGs, each DRG stimulated by one lead 104. These four DRGs are located on three levels, wherein two DRGs are stimulated on the same level. It may be appreciated that any number of DRGs and any combination of DRGs may be stimulated with the stimulation system 100. It may also be appreciated that more than one lead 104 may be positioned so as to stimulate an individual DRG and one lead 104 may be positioned so as to stimulate more than one DRG.

In Fig. 2, each lead 104 is positionable so that its electrodes 106 are able to selectively stimulate the desired target, such as the target DRG, either due to position, electrode configuration, electrode shape, electric field shape, electrode size, stimulation signal parameters, stimulation signal, pattern or algorithm, or any combination of these. Used herein, selective stimulation is stimulation of the target anatomy with little, no or imperceptible stimulation of unwanted anatomies. Such selective stimulation stimulates the targeted neural tissue while excluding untargeted tissue, such as surrounding or nearby tissue. In some cases, the stimulation energy is delivered to the targeted neural tissue so that the energy dissipates or attenuates beyond the targeted tissue or region to a level insufficient to stimulate modulate or influence such untargeted tissue. In some cases, selective stimulation of the DRG describes stimulation of the DRG only, stimulation of the DRG with imperceptible or no stimulation of surrounding tissue, or stimulation of the DRG without stimulation of the ventral root, such as wherein the stimulation signal has an energy below an energy threshold for stimulating a ventral root associated with the target dorsal root while the lead is so positioned.

It may be appreciated that selective stimulation or neuromodulation concepts described herein may be applied in a number of different configurations unilateral (on or in one root ganglion on a level), bi-lateral (on or intwo root ganglion on the same level), unilevel (one or more root ganglion on the same level) or multi-level (at least one root ganglion is stimulated on each of two or more levels) or combinations of the above including stimulation of a portion of the sympathetic nervous system and one or more dorsal root ganglia associated with the neural activity or transmission of that portion of the sympathetic nervous system. As such, the systems may be used to create a wide variety of stimulation control schemes, individually or overlapping, to create and provide zones of treatment.

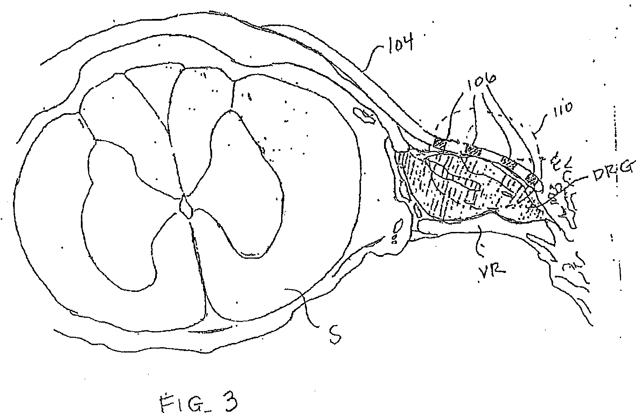

Fig. 3 illustrates an example cross-sectional view of an individual spinal level showing a lead 104 of the stimulation system 100 positioned on a target DRG. The lead 104 is advanced along the spinal cord S to the appropriate spinal level wherein the lead 104 is advanced laterally toward the target DRG. In some instances, the lead 104 is advanced through or partially through a foramen. At least one, some or all of the electrodes 106 are positioned on, near, about, adjacent or in proximity to the DRG. In preferred cases, the lead 104 is positioned so that the electrodes 106 are disposed along a surface of the DRG opposite to the ventral root VR, as illustrated in Fig. 3. It may be appreciated that the surface of the DRG opposite the ventral root VR may be diametrically opposed to portions of the ventral root VR. Such a surface may reside along a variety of areas of the DRG which are separated from the ventral root VR by a distance.

In order to position the lead 104 in such close proximity to the DRG, the lead 104 is appropriately sized and configured to maneuver through the anatomy. In some cases, such maneuvering includes atraumatic epidural advancement along the spinal cord S, through a sharp curve toward a DRG, and optionally through a foramen wherein the distal end of the lead 104 is configured to then reside in close proximity to a small target such as the DRG. Consequently, the lead 104 is significantly smaller and more easily maneuverable than conventional spinal cord stimulator leads.

Referring again to Fig. 3, the electrodes 106 provide a stimulation region which is selective to the target anatomy. In this case, the stimulation region is indicated by dashed line 110, wherein the DRG receives stimulation energy within the stimulation region and the ventral root VR does not as it is outside of the stimulation region. Selective stimulation of the DRG is achieved with the choice of the size of the electrode(s), the shape of the electrode(s), the position of the electrode(s), the stimulation signal, pattern or algorithm or any combination of these. Such selective stimulation stimulates the targeted neural tissue while excluding untargeted tissue, such as surrounding or nearby tissue. In some cases, the stimulation energy is delivered to the targeted neural tissue so that the energy dissipates or attenuates beyond the targeted tissue or region to a level insufficient to stimulate modulate or influence such untargeted tissue. In particular, selective stimulation of tissues, such as the dorsal root, DRG, or portions thereof, exclude stimulation of the ventral root wherein the stimulation signal has an energy below an energy threshold for stimulating a ventral root associated with the target dorsal root while the lead is so positioned.

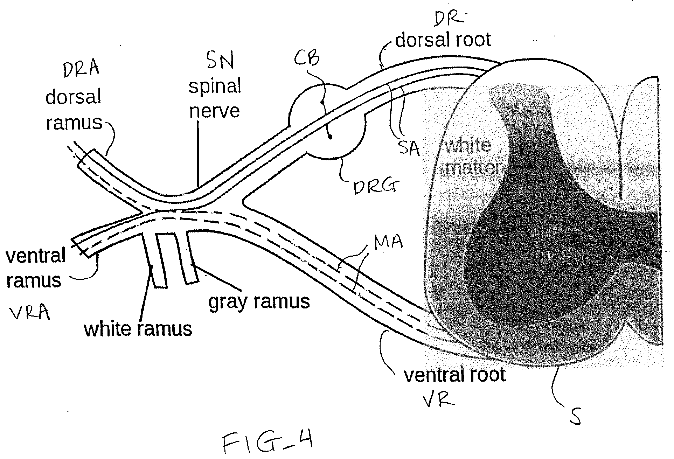

Fig. 4 provides an additional illustration of a pair of spinal nerves extending from the spinal cord S at a spinal level. The pairs are comprised of a dorsal root DR and a ventral root VR. The dorsal root DR carries afferent sensory axons SA (indicated by solid lines) extending from cell bodies CB which are located in the dorsal root ganglion DRG. The ventral root VR carries efferent motor axons MA (indicated by dashed lines). The dorsal root DR and ventral root VR fuse forming a mixed spinal nerve SN which emerges from the spinal column through an opening (intervertebral foramen) between adjacent vertebrae. This is true for all spinal nerves except for the first spinal nerve pair, which emerges between the occipital bone and the atlas (the first vertebra). Thus the cervical nerves are numbered by the vertebra below, except C8, which exists below C7 and above T1. The thoracic, lumbar and sacral nerves are then numbered by the vertebra above. In the case of a lumbarized S1 vertebra (aka L6) or a sacralized L5 vertebra, the nerves are typically still counted to L5 and the next nerve is S 1. Humans have 31 pairs of spinal nerves, each roughly corresponding to a segment of the vertebral column: 8 cervical spinal nerve pairs (C1-C8), 12 thoracic pairs (T1-T12), 5 lumbar pairs (L1-L5), 5 sacral pairs (S1-S5) and 1 coccygeal pair.

The mixed spinal nerve SN carries motor, sensory, and autonomic signals between the spinal cord and the body. Outside the vertebral column, the nerve divides into branches. The dorsal ramus DRA contains nerves that serve the dorsal portions of the trunk carrying visceral motor, somatic motor and somatic sensory information to and from the skin and muscles of the back (epaxial muscles). The ventral ramus VRA contains nerves that serve the remaining ventral parts of the trunk and the upper and lower limbs (hypaxial muscles) carrying visceral motor, somatic motor and sensory information to and from the ventrolateral body surface, structures in the body wall and the limbs. The meningeal branches (recurrent meningeal or sinuvertebral nerves) branch from the spinal nerve and re-enter the intervertebral foramen to serve the ligaments, dura, blood vessels, intervertebral discs, facet joints and periosteum of the vertebrae. The rami communicantes (white ramus and gray ramus) contain autonomic nerves that serve visceral functions carrying visceral motor and sensory information to and from the visceral organs.

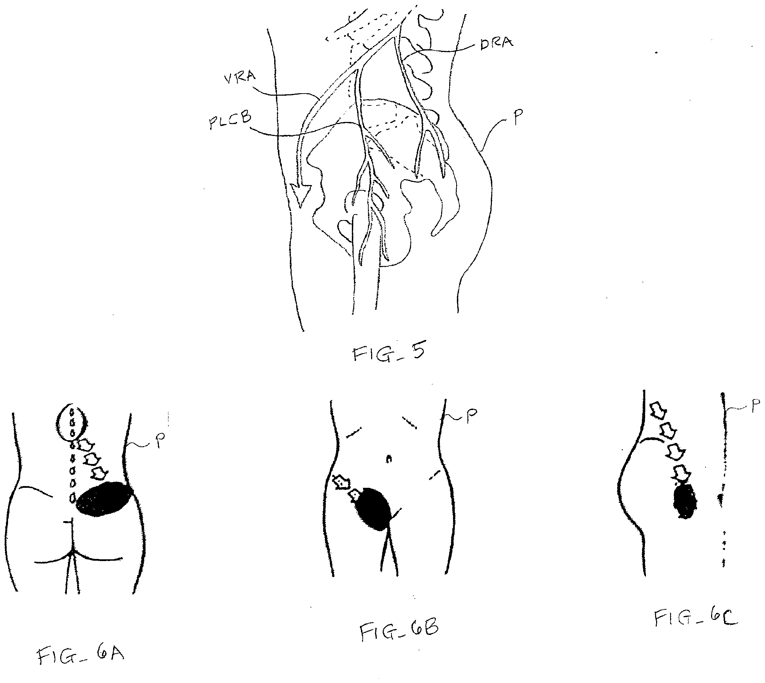

Fig. 5 illustrates an example distribution of spinal nerve branches, such as at T12 or L1, within a patient P. The T12 and L1 spinal nerves emerge at the level of the thoracolumbar junction TLJ and follow a similar course. The dorsal ramus DRA is shown extending posteriorly, down toward the low back, and the ventral ramus VRA is shown extending anteriorly. The ventral ramus VRA supplies the skin of the lower abdomen, the medial aspect of the upper thigh, and the labia majora or the scrotum, the lower part of the rectus abdominis and transversus abdominis muscles, and the pubis. The ventral ramus VRA also branches into a perforating lateral cutaneous branch PLCB which, in this example, emerges above the greater trochanter and supplies the skin of the upper lateral part of the thigh.

Referred pain from the thoracolumbar junction TLJ is felt in the cutaneous distribution of these nerves, the skin and subcutaneous tissues being the site of reflex cellulalgia. However, the pain is felt as deep pain. Figs. 6A-6C illustrate areas of referred pain, as indicated by shading. Fig. 6A illustrates low back pain (dorsal ramus DRA). Fig. 6B illustrates pseudovisceral pain and groin pain (ventral ramus VRA). Fig. 6C illustrates pseudotrochanteric pain (perforating lateral cutaneous branch PLCB). Usually, the cause of such referred pain is painful minor intervertebral dysfunction of a thoracolumbar junction TLJ segment, most often T12/L1, sometimes T11/T12 or L1/L2 (more rare is the T10/T11 level).

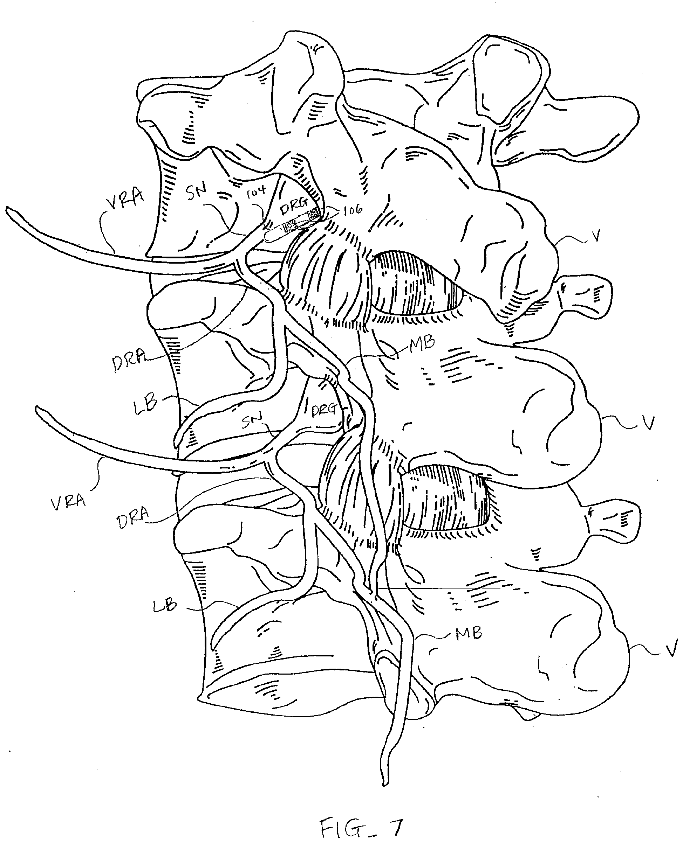

Fig. 7 illustrates spinal nerve branches extending from the vertebral column. Three vertebrae V are illustrated forming a portion of the vertebral column. Each dorsal root ganglion DRG is shown residing within a foramen, an opening between adjacent vertebrae. Beyond the DRG, the mixed spinal nerve SN emerges and divides into branches, the ventral ramus VRA and the dorsal ramus DRA. As shown, each dorsal ramus DRA branches into at least a lateral branch LB and a medial branch MB. The medial branches innervate the facet joints which are often involved in TLJ syndrome and referred pain to the low back.

### Treatment of Pain in the Low Torso

The systems, methods and devices provided herein are able to treat patients suffering from TLJ syndrome or other conditions causing pain in the low torso, such as the low back, hip, groin or other areas. Such systems, methods and devices involve neuromodulating a dorsal root ganglion and/or other neural anatomies to relieve or eliminate the pain sensations. As mentioned previously, in most cases, neuromodulation comprises stimulation, however it may be appreciated that neuromodulation may include a variety of forms of altering or modulating nerve activity by delivering electrical or pharmaceutical agents directly to a target area. Descriptions herein will be provided in terms of stimulation and stimulation parameters.

Referring again to Fig. 7, a lead 104 is shown positioned so as to selectively stimulate the DRG. In this case, the lead 104 is positioned so that at least one of the electrodes 106 is adjacent the DRG. Such placement at spinal level L2 or L3 is particularly suitable for treatment of pain in the lower torso, such as pain associated with the thoracolumbar junction TLJ, low back pain, pseudovisceral pain and groin pain, hip pain and pseudotrochanteric pain, to name a few. However, it may be appreciated that such pain can be treated by such placement of leads 104 at spinal levels T10, T11, T12, L1, L2, L3, L4 and/or L5 due to communications between the lumbar spinal nerves. In any case, a variety of stimulation parameters (electrode selection, amplitude, frequency, pulse width) can be selected to most effectively treat the pain. In some cases, a massaging sensation is generated. Such a massaging sensation differs from a feeling of paresthesia. In some cases, the massaging sensation mimics the feeling of pressure, rubbing, fibrillating or kneading of a muscle. Such a sensation is in contrast to paresthesia which typically has a feeling of burning, prickling, itching, tingling, or 'pins and needles' feeling. In some cases, the massaging sensation is located in the low back. In particular, in some cases, the massaging sensation is felt in the supra-axial muscles, such as the m. multifidus, m. longissimus, and/ or m. iliocostalis. It may be appreciated that in some cases the massaging sensation is felt in other anatomies, such as the hip or groin. In particular, in some cases, the massaging sensation is felt in the infra-axial muscles, such as m. psoas major and/or m. psoas minor. It may also be appreciated that, in some cases, the massaging sensation is felt in the iliopsoas, m. quadratus lumborum, thoracolumbar fascia, m. intertransversarii lumborum or a combination of these, to name a few. Referring back to Fig. 7, in this case, at least a portion of the dorsal root ganglion is stimulated by at least one electrode 106 disposed along the lead 104 to generate such a massaging sensation in a correlated muscle of the lower torso. Such correlation is dictated by innervation by motor and DRG neurons. Spinal segments of motor neurons innervating a muscle are the same as those of DRG neurons projecting afferent fibers from the muscle. Myotomes of lumbar spinal muscles differ from the segmental level of the lumbar spinal site from which the muscle innervation originates. The segmental innervation patterns of the efferent and afferent nervous system are synchronized, showing a non-metameric pattern. In at least one study, at L5, neurons labeled with fluorescent neurotracer were prominent in DRG L3 for the lamina, L2 for the spinous process, L2 for the back muscle fascia and L1 for the skin. Dorsal elements are therefore innervated by neurons in more rostral DRG. Muscles originating from the L5 vertebrae are innervated by motor and DRG neurons predominately in the L2 and L3 spinal levels. Thus, DRGs on various spinal levels have differing innervation patterns. Consequently, stimulation of particular DRGs, such as on spinal levels L2 or L3, are able to generate the massaging sensation in particular muscles with stimulation parameter values that differ from those used on other spinal levels. Stereoscopically, the peripheral innervation territory of a lumbar DRG is conical, with the apex at the ganglion and the base circumference located on the dermatome. The lumbar spine itself is involved in the conical innervation territories of DRG.

In some cases, frequency of the stimulation signal is adjusted to generate the desired massaging sensation in the patient. For example, in some cases, one or more signal parameters are adjusted to produce undesired muscle contractions. This indicates the location of the stimulation and the muscles or body region that is affected by the stimulation. The one or more signal parameters are then readjusted to produce a massaging sensation instead of the undesired muscle contractions. Typically, stimulation at a frequency of less than or equal to approximately 10 Hz, 5 Hz, 4 Hz, 3 Hz, 2 Hz, 1 Hz, 4-5 Hz, 4-10 Hz generates undesired muscle contractions. Adjustment of the frequency to a value such as approximately at least 16 Hz, 16-100 Hz, 20 Hz, 30, Hz, 40 Hz, 50 Hz, 20-50 Hz, 20-30 Hz, 20-40 Hz, 30-40 Hz, 30-50 Hz, 40-50 Hz generates the massaging sensation which is desired by the patient to treat the pain. In other words, once the muscle contractions are detected, the frequency can be increased to transition the undesired muscle contractions to the massaging sensation which treats the pain. A programmer (e.g., 122) can be used to increase the frequency in response to an input from a user or automatically in response to the muscle vibrations being detected. In some cases, the amplitude of the stimulation signal is approximately less than or equal to 2000 µA, more particularly approximately less than or equal to 1000 µA. In some cases, the pulse width of the stimulation signal is approximately 40-300 msec, 200 msec, 300 msec, or 200-300 msec. In some cases pulse width is reduced from a level which provides stimulation sensations in the groin to a level which eliminates stimulation sensations in the groin or reduces such sensations so that they are imperceptible or not noticeable.

The aforementioned undesired muscle contractions can be detected in various different manners. In certain cases, the patient verbally informs a clinician or physician when the patient experiences the undesired muscle contractions. In other cases, the patient presses a button or other input on a programmer (e.g., 122) or other device when the patient experiences the undesired muscle contractions. Either way, patient feedback is used to identify when the muscle contractions occur, indicative of an electrode (being used to deliver the stimulation that causes the muscle contractions) being positioned near the dorsal root ganglion associated with the pain that is to be treated. In further cases, the clinician or physician can use their hand to feel when the undesired muscle contractions occur. A sensor is used to sense when the undesired muscle contractions occur. For example, one or more surface electrodes can provide such a sensor, wherein the surface electrode(s) are used to obtain a surface electromyograph (EMG) from which muscle contractions are detectable by visual inspection of a displayed EMG, or using hardware, software, and/or firmware, or combinations thereof. For another example, needle electrodes can be inserted through the skin to muscle tissue to obtain an intramuscular EMG from which muscle contractions are similarly detectable by visual inspection of a displayed EMG, or using hardware, software, and/or firmware, or combinations thereof. Alternative types of surface or subcutaneous (SubQ) sensors that can be used to detect muscle contractions include accelerometers and vibratory transducers, but are not limited thereto. Such sensors can be connected to a programmer (e.g., 122) or to a trial neurostimulator (TNS) that is used during a lead implant procedure and/or during a test period prior to chronic implantation of the IPG 102. Such a TNS could include components similar to those described above with reference to the Fig. 1B, and thus need not be separately described in further detail. Where a TNS, a programmer, or other device is connected to a sensor (e.g., EMG electrodes or an accelerometer) that is used to detect muscle contractions, such a device can include a display for displaying a signal indicative of the sensed muscle contractions. Alternatively, or additionally, such a device can include hardware, software, and/or firmware, or combinations thereof, that can be used to analyze a sensed signal to determine whether and when the muscle contractions are detected.

In one case, the programmer 122 is configured to cause testing, by the implantable neurostimulator, of a plurality of different combinations of the electrodes 106 to identify which combination of the electrodes results in undesired muscle contractions when used to selectively stimulate at least a portion of the dorsal root ganglion at a first frequency. Additionally, the programmer 122 is configured to increase the first frequency to a second frequency to transition the undesired muscle contractions to the massaging sensation which treats the pain, after the combination of the electrodes which results in the undesired muscle contractions is identified. The first frequency is less than or equal to approximately 10Hz, and the second frequency is at least 16Hz. As mentioned above, a sensor can be configured to detect the undesired muscle contractions.

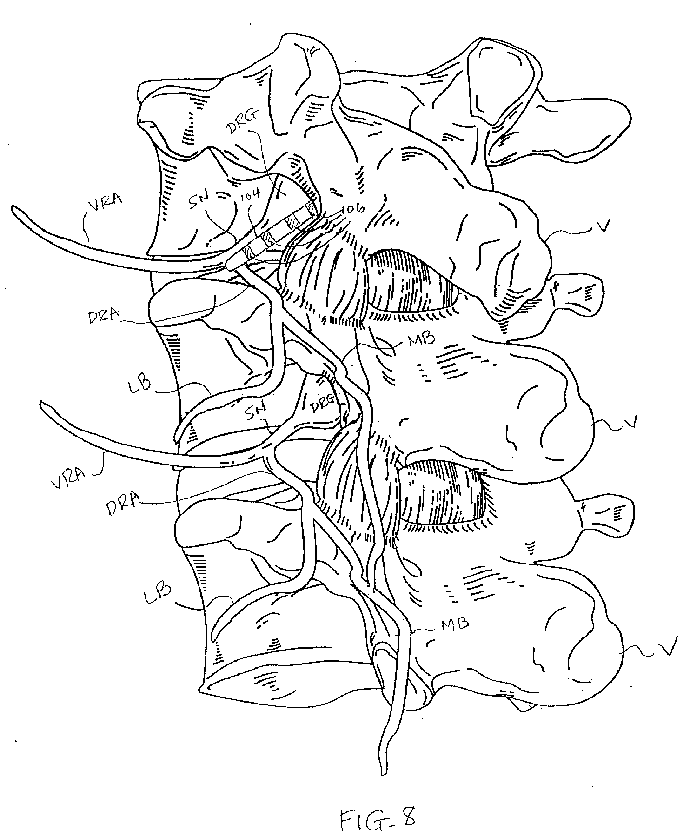

In other cases, the lead 104 is positioned so as to selectively stimulate other target tissues, such as at least a portion of the mixed spinal nerve SN, the ventral ramus VRA, the dorsal ramus DRA, the lateral branch LB, the medial branch MB, the intermediate branch IB, and/or the rami communicantes (white ramus and/or gray ramus). In some cases, such stimulation of other target tissue is achieved in combination with selective stimulation of at least a portion of the associated DRG. Selective stimulation of one or more of such branches at spinal levels T10, T11, T12, L1, L2, L3, L4 and/or L5 is particularly suitable for treatment of pain in the lower torso, such as pain associated with the thoracolumbar junction TLJ. Fig. 8 illustrates an optional lead 104 placement wherein the distal end of the lead 104 is extended through the foramen so as to position at least one electrode near the mixed spinal nerve SN, so as to selectively stimulate the mixed spinal nerve SN. In this case, stimulation parameters (particularly electrode 106 selection) may be varied to selectively stimulate the mixed spinal nerve, the DRG or both. Such stimulation excludes direct stimulation of the ventral root which would lead to undesired motor effects, such as undesired or uncomfortable muscle contraction. In some instances, when the lead 104 is placed as in Fig. 8, stimulation parameters may also be varied to selectively stimulate portions of the dorsal ramus DRA and/or ventral ramus VRA while excluding direct stimulation of the ventral root. In some cases, when stimulating one or a combination of DRG, mixed spinal nerve, dorsal ramus DRA and ventral ramus VRA, a massaging sensation is generated. Such a massaging sensation differs from a feeling of paresthesia as mentioned above. In some cases, when stimulating a portion of the dorsal rami, the massaging sensation is located in the low back, such as in the supra-axial muscles, such as m. multifidus, m. longissimus, and/ or m. iliocostalis. The m. multifidus in particular is a muscle that is targeted in treating low back pain. The multifidus muscle is comprised of a number of fleshy and tendinous fasciculi which fill up the groove on either side of the spinous processes of the vertebrae, from the sacrum to the axis. The multifidus is a very thin muscle. Deep in the spine, it spans three joint segments and works to stabilize the joints at each segmental level. The stiffness and stability makes each vertebra work more effectively, and reduces the degeneration of the joint structures. It may be appreciated that in some cases the massaging sensation is felt in other anatomies, such as the hip or groin. In some cases, when stimulating the ventral rami, the massaging sensation is located in the infra-axial muscles, such as m. psoas major and/or m. psoas minor. Muscles originating from the transverse process of the spine, such as m. intertransversarii lumborum or m. quadratus lumborum, are supplied by nerve branches either from the dorsal or ventral rami of spinal nerves and are therefore affected by stimulation of these nerve branches. It may be appreciated that at least a portion of the iliopsoas and/or thoracolumbar fascia can be affected by stimulation of an associated spinal nerve. Thus, when at least a portion of the mixed spinal nerve SN, dorsal ramus DRA or ventral ramus VRA are stimulated, the massaging sensation is caused by stimulation of motor nerves therein. Recall, the mixed spinal nerve SN, dorsal ramus DRA and ventral ramus VRA carry both motor and sensory neurons. Likewise, motor and DRG neurons innervate muscles in the lower torso as described above.

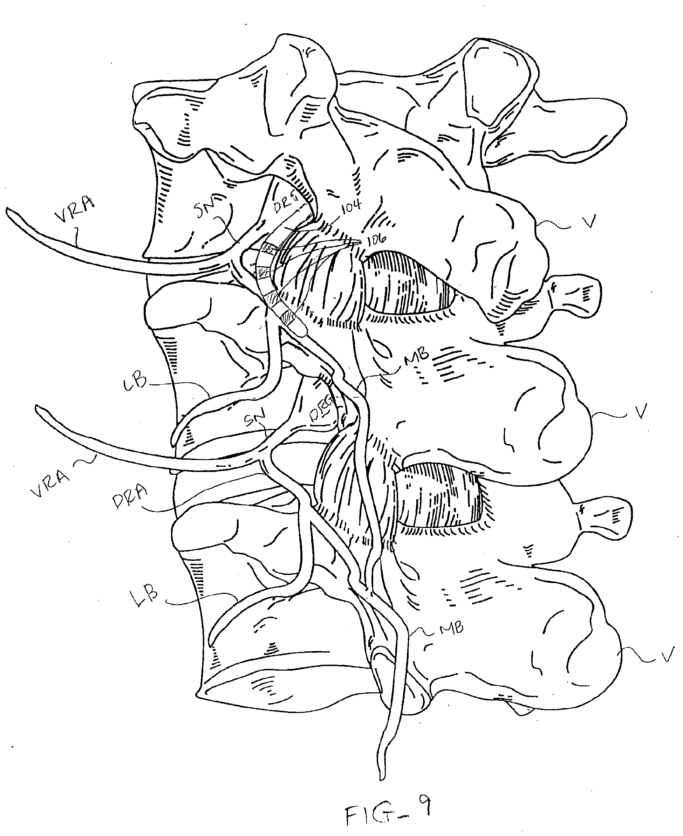

In some cases, the lead 104 is advanced further through the foramen so that at least one electrode 106 is positioned along a portion of the dorsal ramus DRA, such as along the lateral branch LB, the intermediate branch IB and/or the medial branch MB. It may be appreciated that the medial branch MB includes articular branches to the zygopophyseal joints and the periosteum of the vertebral arch. Fig. 9 illustrates a case wherein the lead 104 is advanced so that at least one electrode 106 is positioned along the medial branch MB of the dorsal ramus DRA. Such placement is particularly suitable for treatment of low back pain, particularly back pain associated with the medial branch MB and/or associated with the thoracolumbar junction TLJ. Stimulation parameters can be varied (particularly electrode selection) to selectively stimulate portions of the medial branch MB or any nearby nerve tissue (such as portions of the lateral branch LB, intermediate branch IB or mixed spinal nerve SN, to name a few, without direct stimulation of the ventral nerve. When stimulating one or a combination of these nerve tissues, a massaging sensation is generated. Such a massaging sensation differs from a feeling of paresthesia, as mentioned previously. It may be appreciated that in other cases the lead 104 of Fig. 9 is advanced further along the dorsal ramus DRA so as to stimulate particular branches that are further from the foramen.

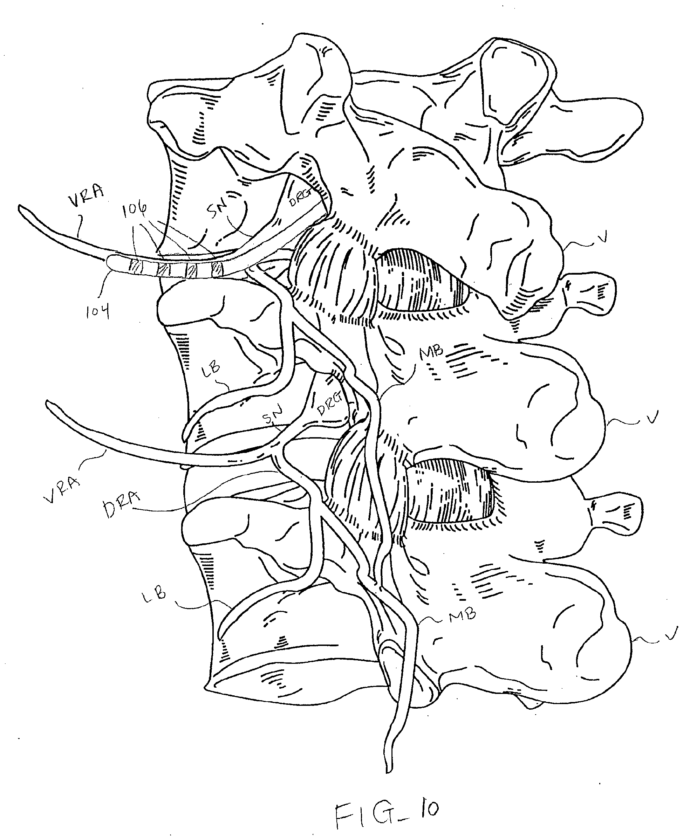

Fig. 10 illustrates a case wherein the lead 104 is advanced further through the foramen so that at least one electrode 106 is positioned along a portion of the ventral ramus VRA. Such placement is particularly suitable for treatment of pseudovisceral pain and groin pain, hip pain and pseudotrochanteric pain, such as when associated with the thoracolumbar junction TLJ. To treat such pain, a variety of stimulation parameters (electrode selection, amplitude, frequency, pulse width) can be selected to most effectively treat the pain. In some cases, a massaging sensation is generated. As mentioned, such a massaging sensation differs from a feeling of paresthesia.

Advancement of the lead 104 as in Fig. 9 and Fig. 10 provides stimulation directly to the target spinal anatomies beyond the foramen. In some of these cases, frequency of the stimulation signal is adjusted to generate the desired massaging sensation in the patient. Typically, stimulation at a frequency of less than or equal to approximately 10 Hz, 5 Hz, 4 Hz, 3 Hz, 2 Hz, 1 Hz, 4-5 Hz, 4-10 Hz generates undesired muscle contraction. Adjustment of the frequency to a value such as approximately at least 16 Hz, 16-100 Hz, 20 Hz, 30, Hz, 40 Hz, 50 Hz, 20-50 Hz, 20-30 Hz, 20-40 Hz, 30-40 Hz, 30-50 Hz, 40-50 Hz generates the massaging sensation which is desired by the patient to treat the pain. In some cases, the amplitude of the stimulation signal is approximately less than or equal to 2000 µA, more particularly approximately less than or equal to 1000 µA. In some cases, the pulse width of the stimulation signal is approximately 40-300 msec, 200 msec, 300 msec or 200-300 msec.

It may be appreciated that, in some cases, the massaging sensation and/or pain relief described herein is achievable with the methods, systems and devices described herein in patients having morphologic changes to their nerve anatomy due to chronic pain, disease, various conditions or other factors. In other words, the morphologic changes to the nerve anatomy of such patients causes such patients to react differently than if such patients were normal, healthy and/or pain-free. For example, in some cases, the massaging sensation resulting from selective stimulation of a DRG in the area of the TLJ is felt in a patient suffering from TLJ syndrome and is not felt in a patient that is pain-free or is not suffering from TLJ syndrome. Likewise, in some cases, the massaging sensation and/or pain relief described herein is achievable using the methods, systems and devices described herein on specific spinal levels wherein such effects are not generated on other spinal levels. In particular, dorsal root ganglions differ in anatomical makeup and/or neural networking depending on spinal level; consequently, differing effects may be achieved. In some cases, spinal levels L2 and L3 are particularly suitable for generation of the massaging sensation.

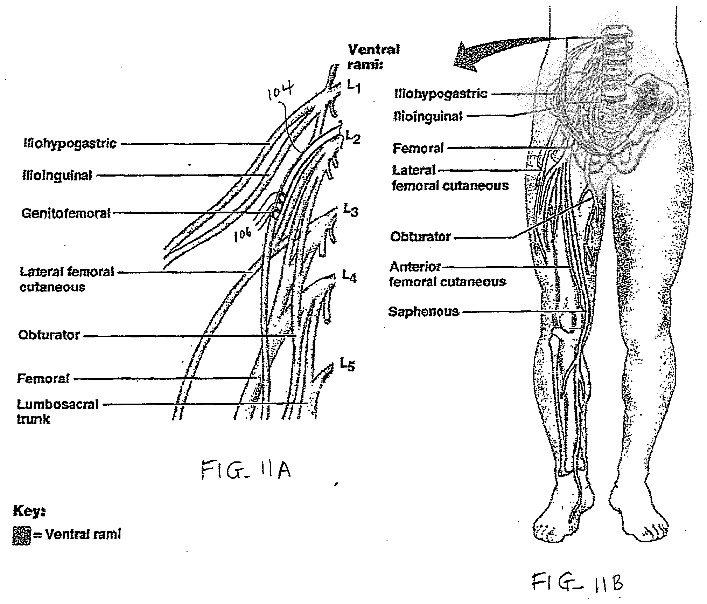

It may be appreciated that the methods, systems and devices may be used to target other peripheral nerves in the low torso or low back area. For example, in some cases, the systems and devices are used to selectively stimulate portions of the lumbar plexus. Referring to Figs. 11A-11B, the lumbar plexus is a nervous plexus in the lumbar region of the body which forms part of the lumbosacral plexus. As shown in Fig. 11A, it is formed by the ventral divisions of the first four lumbar nerves (L1-L4) and from contributions of the subcostal nerve (T12), which is the last thoracic nerve. Additionally, the ventral rami of the fourth lumbar nerve pass communicating branches, the lumbosacral trunk, to the sacral plexus. As shown in Fig. 11B, the nerves of the lumbar plexus pass in front of the hip joint and mainly support the anterior part of the thigh. The plexus is formed lateral to the intervertebral foramina and pass through psoas major. Its smaller motor branches are distributed directly to psoas major, while the larger branches leave the muscle at various sites to run obliquely downward through the pelvic area to leave the pelvis under the inguinal ligament, with the exception of the obturator nerve which exits the pelvis through the obturator foramen.

In one case, the lead 104 is advanced from lumbar segment L2 and positioned within the lumbar plexus, as shown in Fig. 11A. Here, the lead 104 is positioned along the genitofemoral nerve. It may be appreciated that the lead 104 may similarly be positioned along any of the nerves of the lumbar plexus. Referring again to Fig. 11A, the lead 104 is positioned so as to selectively stimulate a portion of the lumbar plexus. Selective stimulation is achieved with the choice of the size of the electrode(s), the shape of the electrode(s), the position of the electrode(s), the stimulation signal, pattern or algorithm, or any combination of these. Such selective stimulation stimulates the targeted neural tissue while excluding untargeted tissue, such as surrounding or nearby tissue. In some cases, the stimulation energy is delivered to the targeted neural tissue so that the energy dissipates or attenuates beyond the targeted tissue or region to a level insufficient to stimulate modulate or influence such untargeted tissue, such as nerves leading to other regions of the leg. In some cases, methods and devices to selectively stimulate any of the nerves of the lumbar plexus are specific to the anatomy of the nerves of the lumbar plexus and/or the condition treated. In particular, the stimulation signal parameters may include a pulse width, frequency, amplitude or a combination of these which selectively stimulates the genitofemoral nerve or any other nerve in the region while excluding or minimizing the stimulation of surrounding or other anatomies.

The lead 104 may be advanced along portions of the lumbar plexus so as to desirably position the electrodes 106 along the target nerve. In order to achieve such positioning, the lead 104 is appropriately sized and configured to maneuver through the local anatomy. Consequently, the lead 104 is significantly smaller and more easily maneuverable than conventional spinal cord stimulator leads. In some cases, the devices used include specialized delivery devices for delivering and positioning the lead near the nerves of the lumbar plexus, such as the genitofemoral nerve (as illustrated) or the iliohypogastric nerve, the ilioinguinal nerve, the lateral femoral cutaneous nerve, the obturator nerve, the femoral nerve or the lumbosacral trunk. The lead may also have particular features, such as small diameter and high flexibility which enable desirable placement and reduced migration in the area of the lumbar plexus.

It may be appreciated that the lead 104 may be advanced to portions of the lumbar plexus or other targets within or beyond the lumbar plexus via atraumatic epidural advancement along the spinal cord S and exiting through a lumbar foramen. However, it may also be appreciated that these targets may be reached by other access routes, including direct access through the skin to the target site, such as in directly into the leg. In some cases, the target nerve is accessed with the use of a needle under guided visualization, such as guided ultrasound. The lead 104 is advanced through the needle so as to position at least one of the electrodes 106 near or on the portion of the nerve to receive the stimulation. It may be appreciated that in some cases, at least one of the electrodes 106 is positioned within the nerve. In some cases, the lead 104 is so positioned with the use of an introducer which has a stiffness suitable for advancement through the tissue near the target nerve anatomy. Likewise, in some cases, the lead is tunneled through the tissue with the use of one or more dilators. It may also be appreciated that in some cases the lead 104 is implanted via conventional surgical methods, or using other endoscopic or laparoscopic techniques.

### Treatment of Discogenic Pain

It may be appreciated that the methods, systems and devices may be utilized on any spinal level and is not limited to the spinal levels associated with TLJ syndrome. It may also be appreciated that the methods, systems and devices may be used to treat other diseases and conditions and is not limited to treating TLJ syndrome or any of the specific areas of pain enumerated herein. For example, the methods, systems and devices may be utilized to treat primary discogenic pain, such as of the lumbar spine. Such pain is often evident in both post-laminotomy syndrome (PLS) and degenerative disc disease (DDD). The principle location of this pain is in the axial distribution of the low back and has often proven to be a challenging target for conventional intraspinal neurostimulation. Typically, the bilateral L2 gray ramus communicans nerves (GRC) are involved in the transmission of discogenic nociceptive pain. Thus, in some cases, treatment of such pain involves stimulation of at least a portion of the gray ramus communicans nerves.

The intervertebral discs are soft tissue structures with an outer annulus of fibrous tissue and an inner core (or nucleus) of softer tissue referred to as the nucleus pulposus situated between the vertebra. Innervation of the intervertebral discs stem both from sympathetic afferents as well as somatic afferent nerves. The somatic nerves course through a normal pathways through the sensory nervous system (through the DRG) and on to the central nervous system. The sympathetic afferent nerves innervating the intervertebral discs can course through the sinuvertebral nerve that enters the central nervous system via the L2 dorsal root ganglion (DRG). Thus, the L2 dorsal root ganglion is a key target in the treatment of discogenic pain. When disruptions to the disc occur resulting in chronic pain, sympathetic nerve sprouting is noted within the disc demonstrating an enhanced innervation pattern that contributes to the chronic pain condition. By targeting the L2 DRG an enhanced ability to treat discogenic low back pain may be realized. Discogenic pain from disc disruption can be mechanically based with a component of neuropathic pain from the rich sympathetic afferent growth observed. Treatment of the L2 ganglion would potentially be able to treat both the mechanically oriented pain condition as well as the neuropathic component due to the innervation patterns.

### Treatment of Other Portions of the Peripheral Nervous System

As mentioned, in some cases the systems and devices are used to stimulate other portions of the peripheral nervous system. The peripheral nervous system is commonly subdivided into two subsystems, the somatic nervous system and the autonomic nervous system. In some cases, the systems and devices are used to selectively stimulate nerves of the autonomic nervous system. Examples of such target nerves are as follows:

### Nerves of the Autonomic Nervous System

### 1) Head/cranial

a) sympathetic
   - ciliary ganglion: roots (sensory, sympathetic), short ciliary
   - ciliary ganglion: roots (sensory, parasympathetic), short ciliary
   - pterygopalatine ganglion: deep petrosal, nerve of pterygoid canal
b) parasympathetic
   - branches of distribution: greater palatine (inferior posterior nasal branches of greater palatine nerve), lesser palatine, nasopalatine (medial superior posterior nasal branches), pharyngeal
   - submandibular ganglion
   - otic ganglion

### 2) Neck/cervical

a) sympathetic
   - paravertebral ganglia: cervical ganglia (superior, middle, inferior), stellate ganglion
   - prevertebral plexus: cavernous plexus, internal carotid

### 3) Chest/thorax

a) sympathetic
   - paravertebral ganglia: thoracic ganglia
   - prevertebral plexus: cardiac plexus, esophageal plexus, pulmonary plexus, thoracic aortic plexus
   - splanchnic nerves: cardiopulmonary, thoracic
   - cardiac nerves: superior, middle, inferior

### 4) Abdomen/Lumbar

a) sympathetic
   - paravertebral ganglia: lumbar ganglia
   - prevertebral ganglia: celiac ganglia (aorticorenal), superior mesenteric ganglion, inferior mesenteric ganglion
   - prevertebral plexus: celiac plexus, (hepatic, splenic, pancreatic), aorticorenal (abdominal aortic plexus, renal/suprarenal), superior mesenteric (gastric), inferior mesenteric (spermatic, ovarian), superior hypogastric (hypogastric nerve, superior rectal), inferior hypogastric (vesical, prostatic/cavernous nerves of penis, uterovaginal, middle rectal)
   - splanchnic nerves: lumbar splanchnic nerves
b) enteric
   - Meissner's plexus
   - Auerbach's plexus

### 5) Pelvis/sacral

a) sympathetic
   - paravertebral ganglia: sacral ganglia, ganglion impar
   - splanchnic nerves: sacral splanchnic nerves
b) parasympathetic
   - splanchnic nerves: pelvic splanchnic nerves

In some cases, the systems and devices are used to selectively stimulate cranial nerves (CN), such as CN I - Olfactory, CN II - Optic, CN III - Oculomotor, CN IV - Trochlear, CN V - Trigeminal, CN VI - Abducens, CN VII - Facial, CN VIII - Vestibulocochlear, CN IX - Glossopharyngeal, CN X - Vagus, CN XI - Accessory, and CN XII - Hypoglossal. In particular cases, the systems and devices are used to selectively stimulate portions of the trigeminal nerve. Examples of such target portions of the trigeminal nerve are as follows:

### Cranial Nerve: Trigeminal Nerve

### A) Ophthalmic

a) frontal: supratrochlear, supraorbital (lateral branch, medial branch)
b) nasociliary: long ciliary, infratrochlear, posterior ethmoidal, anterior ethmoidal (external nasal, internal nasal), sensory root of ciliary ganglion (ciliary ganglion)
c) lacrimal

### B) Maxillary

a) in meninges: middle meningeal
b) in pterygopalatine fossa: zygomatic (zygomaticotemporal, zygornaticofacial), pterygopalatine (pterygopalatine ganglion), posterior superior alveolar
c) in infraorbital canal: infraorbital nerve: superior alveolar (middle, anterior), internal nasal branches
d) on face: inferior palpebral, external nasal, superior labial (infraorbital plexus)

### C) Mandibular

a) in meninges: meningeal
b) anterior: to muscles of mastication (medial pterygoid/to tensor veli palatini, lateral pterygoid, masseteric, deep temporal), buccal
c) posterior: auriculotemporal (otic ganglion), lingual (submandibular ganglion), inferior alveolar (mylohyoid, mental)

The trigeminal nerve is the largest of the cranial nerves. The trigeminal nerve has three major branches: the ophthalmic nerve, the maxillary nerve, and the mandibular nerve. The ophthalmic and maxillary nerves are purely sensory. The mandibular nerve has both sensory and motor functions. The three branches converge on the trigeminal ganglion (also called the semilunar ganglion or gasserian ganglion), that is located within Meckel's cave and contains the cell bodies of incoming sensory nerve fibers. The trigeminal ganglion contains the cell bodies of sensory fibers incoming from the rest of the body. From the trigeminal ganglion, a single large sensory root enters the brainstem at the level of the pons. Immediately adjacent to the sensory root, a smaller motor root emerges from the pons at the same level. Motor fibers pass through the trigeminal ganglion on their way to peripheral muscles, but their cell bodies are located in the nucleus of the fifth nerve, deep within the pons.

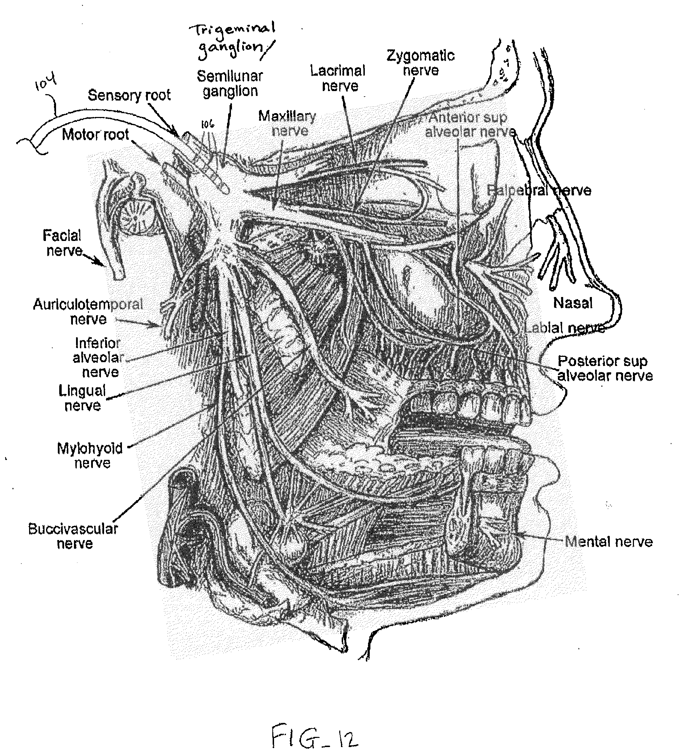

Fig. 12 illustrates a lead 104 positioned so as to selectively stimulate the trigeminal ganglion. Selective stimulation of the trigeminal ganglion is achieved with the choice of the size of the electrode(s), the shape of the electrode(s), the position of the electrode(s), the stimulation signal, pattern or algorithm or any combination of these. Such selective stimulation stimulates the targeted neural tissue while excluding untargeted tissue, such as surrounding or nearby tissue. In some cases, the stimulation energy is delivered to the targeted neural tissue so that the energy dissipates or attenuates beyond the targeted tissue or region to a level insufficient to stimulate modulate or influence such untargeted tissue. In particular, selective stimulation of the trigeminal ganglion excludes stimulation of the nearby motor root wherein the stimulation signal has an energy below an energy threshold for stimulating the motor root while the lead is so positioned. In some cases, methods and devices to selectively stimulate the trigeminal ganglion are specific to the anatomy of the trigeminal ganglion and/or the condition treated. In particular, the stimulation signal parameters may include a pulse width, frequency, amplitude or a combination of these which selectively stimulates the trigeminal ganglion or anatomies within the trigeminal ganglion while excluding or minimizing the stimulation of surrounding or other anatomies. In some cases, the devices include specialized delivery devices for delivering and positioning the lead near the trigeminal ganglion. The lead may also have particular features, such as small diameter and high flexibility which enable desirable placement and reduced migration in the area of the trigeminal ganglion.

The ophthalmic, maxillary and mandibular branches leave the skull through three separate foramina: the superior orbital fissure, the foramen rotundum and the foramen ovale, respectively. The ophthalmic nerve carries sensory information from the scalp and forehead, the upper eyelid, the conjunctiva and cornea of the eye, the nose (including the tip of the nose, except alae nasi), the nasal mucosa, the frontal sinuses, and parts of the meninges (the dura and blood vessels). The maxillary nerve carries sensory information from the lower eyelid and cheek, the nares and upper lip, the upper teeth and gums, the nasal mucosa, the palate and roof of the pharynx, the maxillary, ethmoid and sphenoid sinuses, and parts of the meninges. The mandibular nerve carries sensory information from the lower lip, the lower teeth and gums, the chin and jaw (except the angle of the jaw, which is supplied by C2-C3), parts of the external ear and parts of the meninges. The mandibular nerve carries touch/position and pain/temperature sensation from the mouth. It does not carry taste sensation (chorda tympani is responsible for taste), but one of its branches, the lingual nerve, carries multiple types of nerve fibers that do not originate in the mandibular nerve.

The lead 104 may be advanced beyond the trigeminal ganglion, through the various foramina, including the superior orbital fissure, the foramen rotundum and the foramen ovale, so as to position the electrodes 106 along a desired target nerve within the head or neck. In order to achieve such positioning, the lead 104 is appropriately sized and configured to maneuver through the facial anatomy. Consequently, the lead 104 is significantly smaller and more easily maneuverable than conventional spinal cord stimulator leads.

It may be appreciated that the lead 104 may be advanced to the trigeminal ganglion or other targets within the head, neck and face via atraumatic epidural advancement along the spinal cord S in an antegrade direction. However, it may also be appreciated that these targets may be reached by other access routes, including direct access through the skin to the target site. In some cases, the target nerve is accessed with the use of a needle under guided visualization, such as guided ultrasound. The lead 104 is advanced through the needle so as to position at least one of the electrodes 106 near or on the portion of the nerve to receive the stimulation. It may be appreciated that in some cases, at least one of the electrodes 106 is positioned within the nerve. In some cases, the lead 104 is so positioned with the use of an introducer which has a stiffness suitable for advancement through the tissue near the target nerve anatomy. Likewise, in some cases, the lead is tunneled through the tissue with the use of one or more dilators. It may also be appreciated that in some cases the lead 104 is implanted via conventional surgical methods or using other endoscopic or laparoscopic techniques.

The systems, devices and methods may be used to treat a variety of disorders related to the head, face and neck, such as pain originating in or manifesting in any of these regions. Such pain may result from one or more medical conditions including migraines, headaches, post-traumatic neck pain, post-herpetic neuralgia involving the head, face or neck, myalgia of neck muscles, temporomandibular joint disorder, intracranial hypertension, arthritis, otalgia due to middle ear lesion, maxillary neuralgia, laryngeal pain and sphenopalatine ganglion neuralgia.

In classical anatomy, most sensory information from the face is carried by the trigeminal nerve, but sensation from certain parts of the mouth, certain parts of the ear and certain parts of the meninges is carried by general somatic afferent fibers in cranial nerves VII (the facial nerve), IX (the glossopharyngeal nerve) and X (the vagus nerve). Without exception, however, all sensory fibers from these nerves terminate in the trigeminal nucleus. On entering the brainstem, sensory fibers from V, VII, IX, and X are sorted out and sent to the trigeminal nucleus, which thus contains a complete sensory map of the face and mouth. The spinal counterparts of the trigeminal nucleus (cells in the dorsal horn and dorsal column nuclei of the spinal cord) contain a complete sensory map of the rest of the body. The trigeminal nucleus extends throughout the entire brainstem, from the midbrain to the medulla, and continues into the cervical cord, where it merges with the dorsal horn cells of the spinal cord. The nucleus is divided anatomically into three parts, visible in microscopic sections of the brainstem. From caudal to rostral (i.e., going up from the medulla to the midbrain) they are the spinal trigeminal nucleus, the main trigeminal nucleus and the mesencephalic trigeminal nucleus.

The three parts of the trigeminal nucleus receive different types of sensory information. The spinal trigeminal nucleus receives pain/temperature fibers. The main trigeminal nucleus receives touch/position fibers. The mesencephalic nucleus receives proprioceptor and mechanoreceptor fibers from the jaws and teeth.

The spinal trigeminal nucleus represents pain/temperature sensation from the face. Pain/temperature fibers from peripheral nociceptors are carried in cranial nerves V, VII, IX, and X. On entering the brainstem, sensory fibers are grouped together and sent to the spinal trigeminal nucleus. This bundle of incoming fibers can be identified in cross sections of the pons and medulla as the spinal tract of the trigeminal nucleus, which parallels the spinal trigeminal nucleus itself.

The spinal trigeminal nucleus contains a pain/temperature sensory map of the face and mouth. From the spinal trigeminal nucleus, secondary fibers cross the midline and ascend in the trigeminal lemniscus to the contralateral thalamus. The trigeminal lemniscus runs parallel to the medial lemniscus, which carries pain/temperature information to the thalamus from the rest of the body. Pain/temperature fibers are sent to multiple thalamic nuclei. The central processing of pain/temperature information is markedly different from the central processing of touch/position information.

The main trigeminal nucleus represents touch/position sensation from the face. It is located in the pons, close to the entry site of the fifth nerve. Fibers carrying carry touch/position information from the face and mouth (via cranial nerves V, VII, IX, and X) are sent to the main trigeminal nucleus when they enter the brainstem. The main trigeminal nucleus contains a touch/position sensory map of the face and mouth, just as the spinal trigeminal nucleus contains a complete pain/temperature map.

From the main trigeminal nucleus, secondary fibers cross the midline and ascend in the trigeminal lemniscus to the contralateral thalamus. The trigeminal lemniscus runs parallel to the medial leminscus, which carries touch/position information from the rest of the body to the thalamus. Some sensory information from the teeth and jaws is sent from the main trigeminal nucleus to the ipsilateral thalamus, via the small dorsal trigeminal tract. Thus touch/position information from the teeth and jaws is represented bilaterally in the thalamus (and hence in the cortex). The reason for this special processing is discussed below.

The mesencephalic trigeminal nucleus is not really a nucleus. Rather, it is a sensory ganglion that happens to be embedded in the brainstem. The mesencephalic nucleus is the sole exception to the general rule that sensory information passes through peripheral sensory ganglia before entering the central nervous system. Only certain types of sensory fibers have cell bodies in the mesencephalic nucleus: proprioceptor fibers from the jaw and mechanoreceptor fibers from the teeth. Some of these incoming fibers go to the motor nucleus of V, thus entirely bypassing the pathways for conscious perception. The jaw jerk reflex is an example. Tapping the jaw elicits a reflex closure of the jaw, in exactly the same way that tapping the knee elicits a reflex kick of the lower leg. Other incoming fibers from the teeth and jaws go to the main nucleus of V. As noted above, this information is projected bilaterally to the thalamus. It is available for conscious perception.

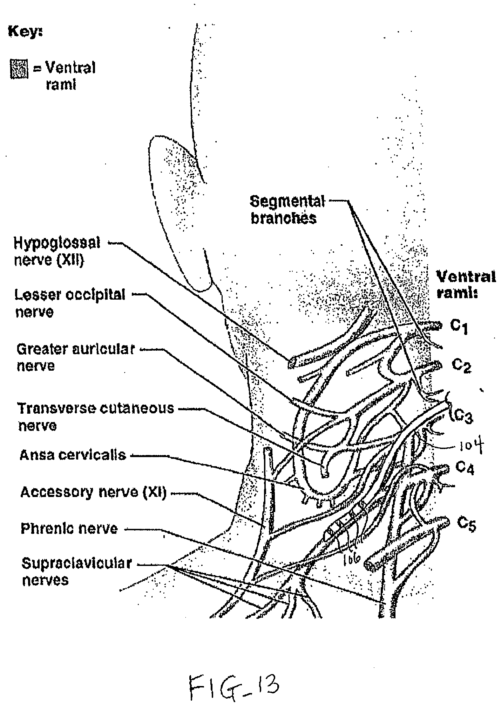

In some cases, the systems and devices are used to selectively stimulate portions of the cervical plexus. Referring to Fig. 13, the cervical plexus is a plexus of the ventral rami of the first four cervical spinal nerves which are located from C1 to *CS* cervical segment in the neck. They are located laterally to the transverse processes between prevertebral muscles from the medial side and vertebral (m.scalenus, m.levator scapulae, m.splenius cervicis) from the lateral side. There is anastomosis with an accessory nerve, hypoglossal nerve and sympathetic trunk. The cervical plexus is located in the neck, deep to stemocleidomastoid. Nerves formed from the cervical plexus innervate the back of the head, as well as some neck muscles. The branches of the cervical plexus emerge from the posterior triangle at the nerve point, a point which lies midway on the posterior border of the stemocleidomastoid. In one case, the lead 104 is advanced from cervical segment C3 and positioned within the cervical plexus, as shown in Fig. 13. Here, the lead 104 is positioned along a nerve leading to the supraclavicular nerves. It may be appreciated that the lead 104 may similarly be positioned along any of the nerves of the cervical plexus. Referring again to Fig. 13, the lead 104 is positioned so as to selectively stimulate a portion of the cervical plexus. Selective stimulation is achieved with the choice of the size of the electrode(s), the shape of the electrode(s), the position of the electrode(s), the stimulation signal, pattern or algorithm, or any combination of these. Such selective stimulation stimulates the targeted neural tissue while excluding untargeted tissue, such as surrounding or nearby tissue. In some cases, the stimulation energy is delivered to the targeted neural tissue so that the energy dissipates or attenuates beyond the targeted tissue or region to a level insufficient to stimulate modulate or influence such untargeted tissue. In some cases, methods and devices to selectively stimulate the cervical plexus are specific to the anatomy of the nerves of the cervical plexus and/or the condition treated. In particular, the stimulation signal parameters may include a pulse width, frequency, amplitude or a combination of these to selectively stimulate the nerves of the cervical plexus while excluding or minimizing the stimulation of surrounding or other anatomies.

The lead 104 may be advanced along portions of the cervical plexus so as to desirably position the electrodes 106 along the target nerve. In order to achieve such positioning, the lead 104 is appropriately sized and configured to maneuver through the anatomy of the neck. Consequently, the lead 104 is significantly smaller and more easily maneuverable than conventional spinal cord stimulator leads. In some cases, the devices include specialized delivery devices for delivering and positioning the lead near the nerves of the cervical plexus. The lead may also have particular features, such as small diameter and high flexibility which enable desirable placement and reduced migration in the area of the cervical plexus.

It may be appreciated that the lead 104 may be advanced to portions of the cervical plexus or other targets within or beyond the cervical plexus via atraumatic epidural advancement along the spinal cord S and exiting through a cervical foramen. However, it may also be appreciated that these targets may be reached by other access routes, including direct access through the skin to the target site. In some cases, the target nerve is accessed with the use of a needle under guided visualization, such as guided ultrasound. The lead 104 is advanced through the needle so as to position at least one of the electrodes 106 near or on the portion of the nerve to receive the stimulation. It may be appreciated that in some cases, at least one of the electrodes 106 is positioned within the nerve. In some cases, the lead 104 is so positioned with the use of an introducer which has a stiffness suitable for advancement through the tissue near the target nerve anatomy. Likewise, in some cases, the lead is tunneled through the tissue with the use of one or more dilators. It may also be appreciated that in some cases the lead 104 is implanted via conventional surgical methods, or using other endoscopic or laparoscopic techniques.

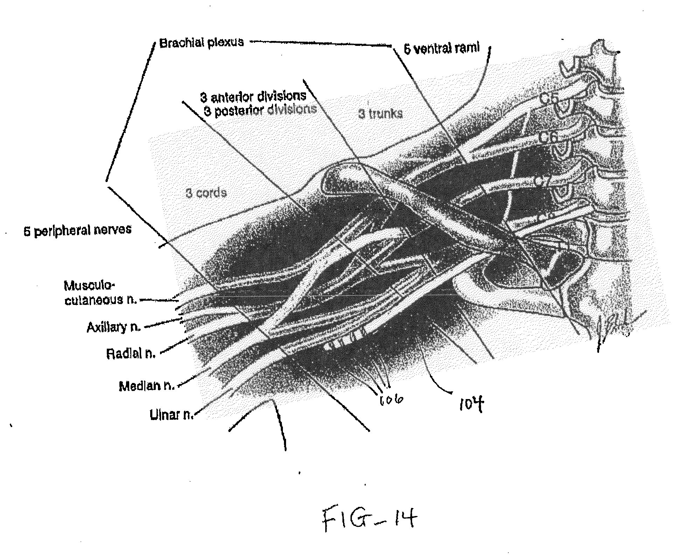

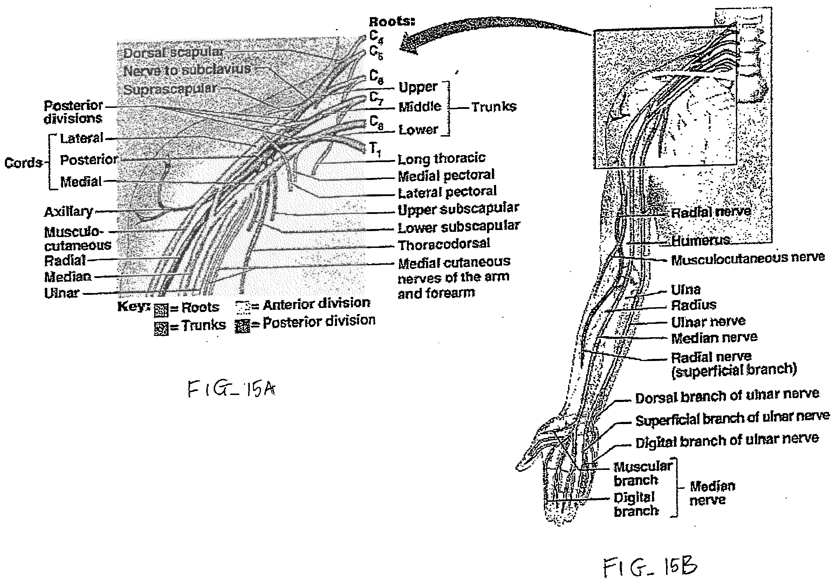

In some cases, the systems and devices are used to selectively stimulate portions of the brachial plexus. Referring to Fig. 14, the brachial plexus is an arrangement of nerve fibers, running from the spine, formed by the ventral rami of the lower four cervical and first thoracic nerve roots (C5-T1). It proceeds through the neck, the axilla (armpit region), and into the arm. Fig. ISA provides another view of the brachial plexus anatomy residing in the shoulder and Fig. 15B illustrates the nerves extending into the arm and hand. In one case, the lead 104 is advanced from cervical segment C8 and positioned within the brachial plexus, as shown in Fig. 14. Here, the lead 104 is positioned along a nerve leading to the ulnar nerve. It may be appreciated that the lead 104 may similarly be positioned along any of the nerves of the brachial plexus. Referring again to Fig. 14, the lead 104 is positioned so as to selectively stimulate a portion of the brachial plexus. Selective stimulation is achieved with the choice of the size of the electrode(s), the shape of the electrode(s), the position of the electrode(s), the stimulation signal, pattern or algorithm, or any combination of these. Such selective stimulation stimulates the targeted neural tissue while excluding untargeted tissue, such as surrounding or nearby tissue. In some cases, the stimulation energy is delivered to the targeted neural tissue so that the energy dissipates or attenuates beyond the targeted tissue or region to a level insufficient to stimulate modulate or influence such untargeted tissue, such as nerves leading to other regions of the arm and hand. In some cases, methods and devices to selectively stimulate the brachial plexus are specific to the anatomy of the nerves of the brachial plexus and/or the condition treated. In particular, the stimulation signal parameters may include a pulse width, frequency, amplitude or a combination of these to selectively stimulate the nerves of the brachial plexus while excluding or minimizing the stimulation of surrounding or other anatomies.

The lead 104 may be advanced along portions of the brachial plexus so as to desirably position the electrodes 106 along the target nerve. In order to achieve such positioning, the lead 104 is appropriately sized and configured to maneuver through the local anatomy. Consequently, the lead 104 is significantly smaller and more easily maneuverable than conventional spinal cord stimulator leads. In some cases, the devices include specialized delivery devices for delivering and positioning the lead near the nerves of the brachial plexus.
The lead may also have particular features, such as small diameter and high flexibility which enable desirable placement and reduced migration in the area of the brachial plexus.

It may be appreciated that the lead 104 may be advanced to portions of the brachial plexus or other targets within or beyond the brachial plexus via atraumatic epidural advancement along the spinal cord S and exiting through a cervical or thoracic foramen. However, it may also be appreciated that these targets may be reached by other access routes, including direct access through the skin to the target site, such as in directly into the arm or hand. In some cases, the target nerve is accessed with the use of a needle under guided visualization, such as guided ultrasound. The lead 104 is advanced through the needle so as to position at least one of the electrodes 106 near or on the portion of the nerve to receive the stimulation. It may be appreciated that in some cases, at least one of the electrodes 106 is positioned within the nerve. In some cases, the lead 104 is so positioned with the use of an introducer which has a stiffness suitable for advancement through the tissue near the target nerve anatomy. Likewise, in some cases, the lead is tunneled through the tissue with the use of one or more dilators. It may also be appreciated that in some cases the lead 104 is implanted via conventional surgical methods, or using other endoscopic or laparoscopic techniques.

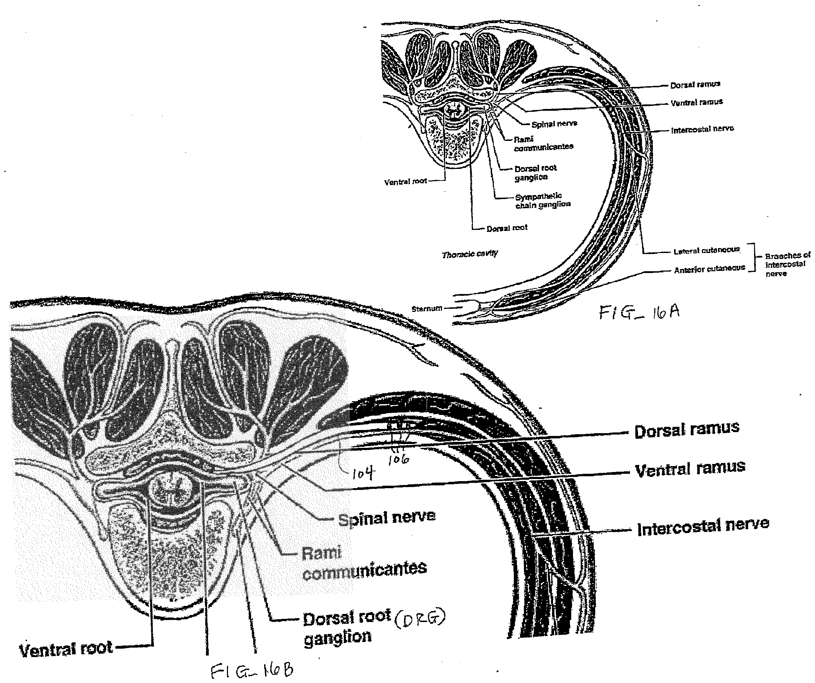

In some cases, the systems and devices are used to selectively stimulate portions of the peripheral nervous system throughout the thoracic region. Fig. 16A provides a cross-sectional view of the thoracic cavity and associated spinal anatomy. As shown, the peripheral nervous system extends to the sympathetic chain ganglions, the dorsal ramus and the ventral ramus. The ventral ramus extends to the intercostal nerves. The intercostal nerves are the anterior divisions (rami anteriores; ventral divisions) of the thoracic spinal nerves from T1 to T1 I. Each nerve is connected with the adjoining ganglion of the sympathetic trunk by a gray and a white ramus communicans. The intercostal nerves are distributed chiefly to the thoracic pleura and abdominal peritoneum and differ from the anterior divisions of the other spinal nerves in that each pursues an independent course without plexus formation. The first two nerves supply fibers to the upper limb in addition to their thoracic branches; the next four are limited in their distribution to the parietes of the thorax; the lower five supply the parietes of the thorax and abdomen. The 7th intercostal nerve terminates at the xyphoid process, at the lower end of the sternum. The 10th intercostal nerve terminates at the umbilicus. The twelfth (subcostal) thoracic is distributed to the abdominal wall and groin.

In one case, illustrated in Fig. 16B, the lead 104 is advanced epidurally along the spinal cord S, through a sharp curve toward a DRG, and through a foramen wherein the distal end of the lead 104 is positioned along an intercostal nerve. It may be appreciated that the lead 104 may similarly be positioned along any of the nerves of the thoracic region. Referring again to Fig. 16B, the lead 104 is positioned so as to selectively stimulate a portion of the intercostal nerve. Selective stimulation is achieved with the choice of the size of the electrode(s), the shape of the electrode(s), the position of the electrode(s), the stimulation signal, pattern or algorithm, or any combination of these. Such selective stimulation stimulates the targeted neural tissue while excluding untargeted tissue, such as surrounding or nearby tissue. In some cases, the stimulation energy is delivered to the targeted neural tissue so that the energy dissipates or attenuates beyond the targeted tissue or region to a level insufficient to stimulate modulate or influence such untargeted tissue, such as nerves leading to other regions of the thoracic cavity or points beyond. In some cases, methods and devices to selectively stimulate the intercostal nerve are specific to the anatomy of the nerves of the thoracic region and/or the condition treated. In particular, the stimulation signal parameters may include a pulse width, frequency, amplitude or a combination of these which selectively stimulates the intercostal nerve while excluding or minimizing the stimulation of surrounding or other anatomies.

The lead 104 may be advanced along portions of the intercostal nerves so as to desirably position the electrodes 106 along the target nerve. In order to achieve such positioning, the lead 104 is appropriately sized and configured to maneuver through the epidural space, through a foramen and within the local anatomy. Consequently, the lead 104 is significantly smaller and more easily maneuverable than conventional spinal cord stimulator leads. In some cases, the devices include specialized delivery devices for delivering and positioning the lead near the nerves of the thoracic region, particularly the intercostal nerve. The lead may also have particular features, such as small diameter and high flexibility which enable desirable placement and reduced migration in the area of the interocostal nerve.

It may be appreciated that the lead 104 may be advanced to portions of the thoracic region or beyond by other access routes, including direct access through the skin to the target site, such as in directly into the torso. In some cases, the target nerve is accessed with the use of a needle under guided visualization, such as guided ultrasound. The lead 104 is advanced through the needle so as to position at least one of the electrodes 106 near or on the portion of the nerve to receive the stimulation. It may be appreciated that in some cases, at least one of the electrodes 106 is positioned within the nerve. In some cases, the lead 104 is so positioned with the use of an introducer which has a stiffness suitable for advancement through the tissue near the target nerve anatomy. Likewise, in some cases, the lead is tunneled through the tissue with the use of one or more dilators. It may also be appreciated that in some cases the lead 104 is implanted via conventional surgical methods or using other endoscopic or laparoscopic techniques.

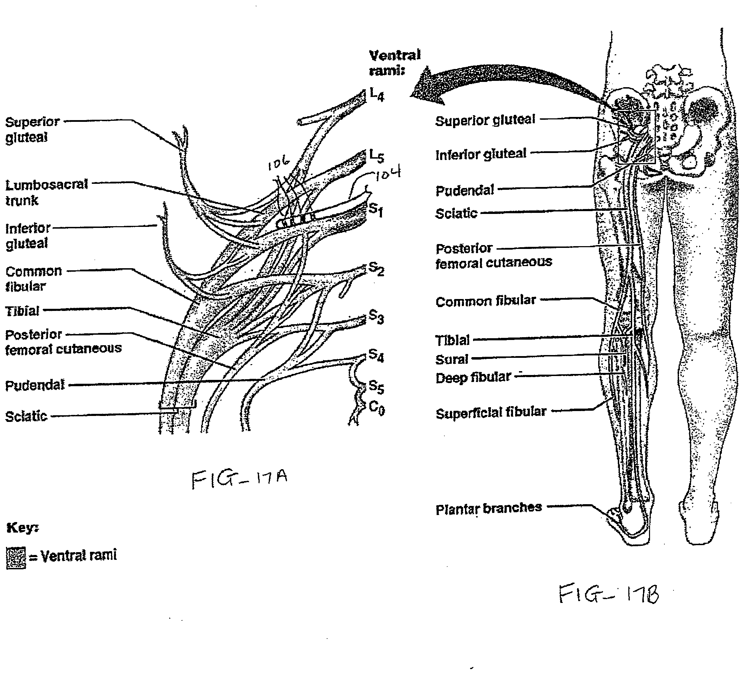

In some cases, the systems and devices are used to selectively stimulate portions of the sacral plexus. Referring to Figs. 17A-17B, the sacral plexus is a nerve plexus which provides motor and sensory nerves for the posterior thigh, most of the lower leg, the entire foot and part of the pelvis. It is part of the lumbosacral plexus and emerges from the sacral vertebrae (S2-S4). The sacral plexus is formed by the lumbosacral trunk, the anterior division of the first sacral nerve and portions of the anterior divisions of the second and third sacral nerves. The nerves forming the sacral plexus converge toward the lower part of the greater sciatic foramen and unite to form a flattened band, from the anterior and posterior surfaces of which several branches arise. The band itself is continued as the sciatic nerve, which splits on the back of the thigh into the tibial nerve and common fibular nerve; these two nerves sometimes arise separately from the plexus, and in all cases their independence can be shown by dissection. Often, the sacral plexus and the lumbar plexus are considered to be one large nerve plexus, the lumbosacral plexus. The lumbosacral trunk connects the two plexuses.

In one case, the lead 104 is advanced from sacral segment SI and positioned within the sacral plexus, as shown in Fig. 17A. Here, the lead 104 is positioned along a nerve leading to the common fibular nerve. It may be appreciated that the lead 104 may similarly be positioned along any of the nerves of the sacral plexus. Referring again to Fig. 17A, the lead 104 is positioned so as to selectively stimulate a portion of the sacral plexus. Selective stimulation is achieved with the choice of the size of the electrode(s), the shape of the electrode(s), the position of the electrode(s), the stimulation signal, pattern or algorithm, or any combination of these. Such selective stimulation stimulates the targeted neural tissue while excluding untargeted tissue, such as surrounding or nearby tissue. In some cases, the stimulation energy is delivered to the targeted neural tissue so that the energy dissipates or attenuates beyond the targeted tissue or region to a level insufficient to stimulate modulate or influence such untargeted tissue, such as nerves leading to other regions of the leg or foot. In some cases, methods and devices to selectively stimulate any of the nerves of the sacral plexus are specific to the anatomy of the nerves of the sacral plexus and/or the condition treated. In particular, the stimulation signal parameters may include a pulse width, frequency, amplitude or a combination of these which selectively stimulates a nerve of the sacral plexus or any other nerve in the region while excluding or minimizing the stimulation of surrounding or other anatomies.

The lead 104 may be advanced along portions of the sacral plexus so as to desirably position the electrodes 106 along the target nerve. In order to achieve such positioning, the lead 104 is appropriately sized and configured to maneuver through the local anatomy. Consequently, the lead 104 is significantly smaller and more easily maneuverable than conventional spinal cord stimulator leads. In some cases, the devices include specialized delivery devices for delivering and positioning the lead near the nerves of the sacral plexus. The lead may also have particular features, such as small diameter and high flexibility which enable desirable placement and reduced migration in the area of the sacral plexus.

It may be appreciated that the lead 104 may be advanced to portions of the sacral plexus or other targets within or beyond the sacral plexus via atraumatic epidural advancement along the spinal cord S and exiting through a sacral foramen. However, it may also be appreciated that these targets may be reached by other access routes, including direct access through the skin to the target site, such as in directly into the leg or foot. In some cases, the target nerve is accessed with the use of a needle under guided visualization, such as guided ultrasound. The lead 104 is advanced through the needle so as to position at least one of the electrodes 106 near or on the portion of the nerve to receive the stimulation. It may be appreciated that in some cases, at least one of the electrodes 106 is positioned within the nerve. In some cases, the lead 104 is so positioned with the use of an introducer which has a stiffness suitable for advancement through the tissue near the target nerve anatomy. Likewise, in some cases, the lead is tunneled through the tissue with the use of one or more dilators. It may also be appreciated that in some cases the lead 104 is implanted via conventional surgical methods or using other endoscopic or laparoscopic techniques.

### Approaching Target Anatomies

As mentioned, any of the target anatomies can be accessed via an epidural approach, wherein the lead is advanced along the spinal cord S, curves laterally away from the midline, is advanced along a nerve root and (for target anatomies beyond the dorsal root ganglion) exits the epidural space through a foramen. In some cases, the epidural approach is an antegrade approach. It may be appreciated that, alternatively, other approaches may be used, such as a retrograde approach or a contralateral approach.

By approaching target anatomies in the peripheral nervous system via an epidural approach, multiple points of mechanical stability are provided to the lead. To begin, the epidural space acts as a lead stabilization pathway. As the spine is a stiff, stable structure, the lead is protected from major movements that would place strain on the lead and tend to propagate migration. In addition, at the point of exiting the foramen, the lead is also stabilized by the bony opening and soft tissue within the foramen. Anchoring near the spine provides a more stable grounding or anchoring of the lead to the anatomy (the spine and adjacent fascia) compared to the soft tissues in the periphery.

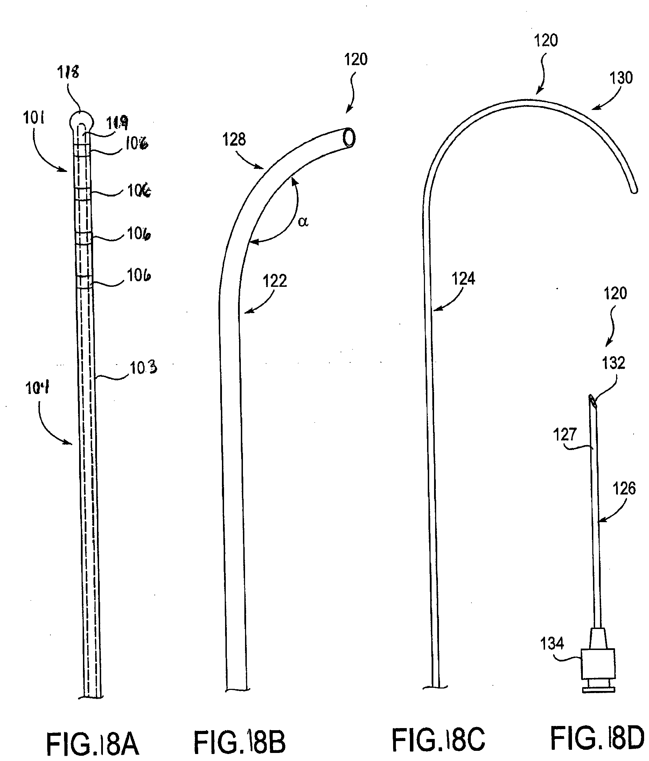

In some cases, the lead is delivered to the dorsal root ganglion and/or targets beyond the foramen in the peripheral nervous system with the use of a delivery system. Figs. 18A-18B illustrate a lead 104 (Fig. 8A) and delivery system 120, including a sheath 122 (Fig. 8B), stylet 124 (Fig. 8C) and introducing needle 126 (Fig. 8D), for such purposes. In this case, the lead 104 comprises a shaft 103 having a distal end 101 and four electrodes 106 disposed thereon. It may be appreciated that any number of electrodes 106 may be present, including one, two, three, four, five, six, seven, eight or more. In this case, the distal end 101 has a closed-end distal tip 118. The distal tip 118 may have a variety of shapes including a rounded shape, such as a ball shape (shown) or tear drop shape and a cone shape. These shapes provide an atraumatic tip for the lead 104 as well as serving other purposes. The lead 104 also includes a stylet lumen 119 which extends toward the closed-end distal tip 118.

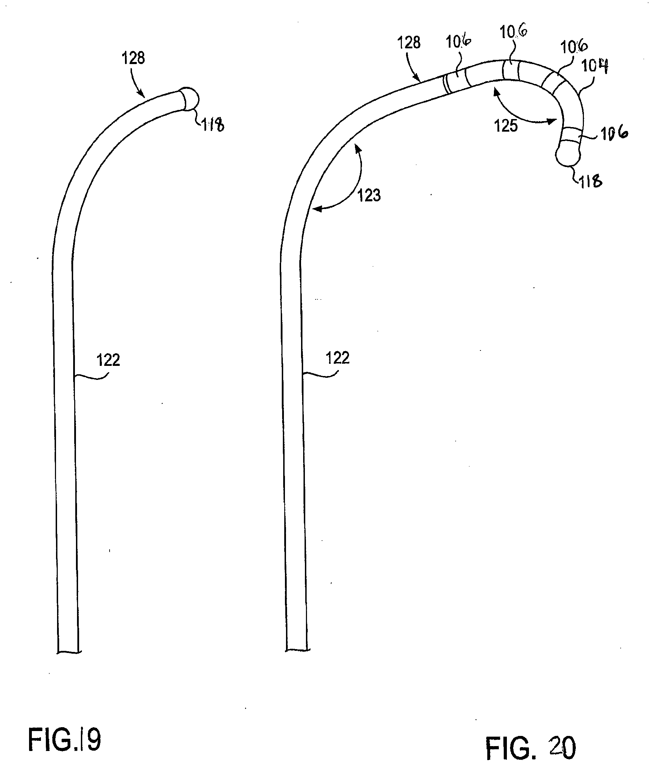

Fig. 18B illustrates a sheath 122. The sheath 122 has a distal end 128 which is pre-curved to have an angle α, wherein the angle α is in the range of approximately 80 to 165 degrees, in this case. The sheath 122 is sized and configured to be advanced over the shaft 103 of the lead 104 until a portion of its distal end 128 abuts the distal tip 118 of the lead 104, as illustrated in Fig. 19. Thus, the ball shaped tip 118 also prevents the sheath 122 from extending thereover. Passage of the sheath 122 over the lead 104 causes the lead 104 to bend in accordance with the precurvature of the sheath 122. Thus, the sheath 122 assists in steering the lead 104 along the spinal column S and toward a target DRG, such as in a lateral direction. It may be appreciated that the angle α may optionally be smaller, such as less than 80 degrees, forming a U-shape or tighter bend.

Referring back to Fig. 18C, a stylet 124 is illustrated. In this case, the stylet 124 has a distal end 130 which is pre-curved so that its radius of curvature is in the range of approximately 2.54 to 12.7 mm (0.1 to 0.5 inches). The stylet 124 is sized and configured to be advanced within the stylet lumen 119 of the lead 104. Typically the stylet 124 extends therethrough so that its distal end 130 aligns with the distal end 101 of the lead 104. Passage of the stylet 124 through the lead 104 causes the lead 104 to bend in accordance with the precurvature of the stylet 124. Typically, the stylet 124 has a smaller radius of curvature, or a tighter bend, than the sheath 122. Therefore, as shown in Fig. 20, when the stylet 124 is disposed within the lead 104, extension of the lead 104 and stylet 124 through the sheath 122 bends or directs the lead 104 through a first curvature 123. Further extension of the lead 104 and stylet 124 beyond the distal end 128 of the sheath 122 allows the lead 104 to bend further along a second curvature 125. When approaching a target DRG, the second curvature allows the laterally directed lead 104 to now curve around toward the target DRG, such as along the nerve root angulation. This two step curvature allows the lead 104 to be successfully positioned so that at least one of the electrodes 106 is on, near, about, adjacent or in proximity to the target DRG, particularly by making a sharp turn along the angle θ. In addition, the electrodes 106 are spaced to assist in making such a sharp turn.

Thus, the lead 104 does not require stiff or torqueable construction since the lead 104 is typically not torqued or steered by itself. The lead 104 is positioned with the use of the sheath 122 and stylet 124 which direct the lead 104 through the two step curvature. This eliminates the need for the operator to torque the lead 104 and optionally the sheath 122 with multiple hands. This also allows the lead 104 to have a lower profile and smaller diameter, as well as a very soft and flexible construction. This, in turn, minimizes erosion, irritation of the neural tissue and discomfort created by pressure on nerve tissue, such as the target DRG and/or other nerves, once the lead 104 is implanted. In addition, such a soft and flexible lead 104 will minimize the amount of force translated to the distal end of the lead 104 by body movement (e.g. flexion, extension, torsion).

Referring back to Fig. 18D, an introducing needle 126 is illustrated. The introducing needle 126 is used to access the epidural space of the spinal cord S. The needle 126 has a hollow shaft 127 and typically has a very slightly curved distal end 132. The shaft 127 is sized to allow passage of the lead 104, sheath 122 and stylet 124 therethrough. In some cases, the needle 126 is 2.108 mm (14 gauge) which is typically the size of epidural needles used to place conventional percutaneous leads within the epidural space. However, it may be appreciated that other sized needles may also be used, particularly smaller needles such as between 1.270 and 1.829 mm (15-18 gauge). Alternatively, non-standardized sized needles may be used.

The needle is atraumatic so as to not damage the sheath 122 when the sheath 122 is advanced or retracted. In some cases, the shaft 127 comprises a low friction material, such as bright hypotubing, made from bright steel (a product formed from the process of drawing hot rolled steel through a die to impart close dimensional tolerances, a bright, scale free surface and improved mechanical properties). Other materials include polytetrafluoroethylene (PTFE) impregnated or coated hypotubing. In addition, it may be appreciated that needles having various tips known to practitioners or custom tips designed for specific applications may also be used. The needle 126 also typically includes a luer fitting 134, such as a Luer-Lok™ fitting, or other fitting near its proximal end. The luer fitting 134 is a female fitting having a tabbed hub which engages threads in a sleeve on a male fitting, such as a syringe. The needle 126 may also have a luer fitting on a side port, so as to allow injection through the needle 126 while the sheath 122 is in the needle 126. In some cases, the luer fitting is tapered to allow for easier introduction of a curved sheath into the hollow shaft 127.

The above described delivery system 120 is used for epidural delivery of the lead 104 through the patient anatomy toward a target DRG or points beyond the foramen, such as the peripheral system. Thus, epidural delivery methods are described herein. In particular, such methods are described and illustrated as an antegrade approach. It may be appreciated that, alternatively, the devices and systems may be used with a retrograde approach or a contralateral approach. Likewise, at least some of the devices and systems may be used with a transforaminal approach, wherein the target is approached from outside of the spinal column. Further, a target may be approached through the sacral hiatus or through a bony structure such as a pedicle, lamina or other structure.

Epidural delivery involves accessing the epidural space. The epidural space is accessed with the use of the introducing needle 126, as illustrated in Fig. 18D. Typically, the skin is infiltrated with local anesthetic such as lidocaine over the identified portion of the epidural space. The insertion point is usually near the midline M, although other approaches may be employed. Typically, the needle 126 is inserted to the ligamentum flavum and a loss of resistance to injection technique is used to identify the epidural space. A syringe 140 is then attached to the needle 126, typically containing air or saline. Traditionally either air or saline has been used for identifying the epidural space, depending on personal preference. When the tip of the needle 126 enters a space of negative or neutral pressure (such as the epidural space), there will be a loss of resistance and it will be possible to inject through the syringe 140. In addition to the loss of resistance technique, real time observation of the advancing needle 126 may be achieved with a portable ultrasound scanner or with fluoroscopy. Likewise, a guidewire may be advanced through the needle 126 and observed within the epidural space with the use of fluoroscopy.

Once the needle 126 has been successfully inserted into the epidural space, the syringe 140 is removed. The stylet 124 is inserted into the lead 104 and the sheath 122 is advanced over the lead 104. The sheath 122 is positioned so that its distal end 128 is near or against the distal tip 106 of the lead 104 causing the lead 104 to follow the curvature of the sheath 122. The stylet 124, lead 104 and sheath 122 are then inserted through the needle 126, into the epidural space and the assembled sheath 122/lead 104/stylet 124 emerges therefrom. The rigidity of the needle 126 straightens the more flexible sheath 122 as it passes therethrough. However, upon emergence, the sheath 122 is allowed to bend along or toward its precurvature. In some cases, the shape memory of the sheath 122 material allows the sheath 122 to retain more than 50% of its precurved shape upon passing through the needle 126. Such bending assists in steering of the lead 104 within the epidural space. This is particularly useful when using a retrograde approach to navigate across the transition from the lumbar spine to the sacral spine. The sacrum creates a shelf that resists ease of passage into the sacrum. The precurved sheath 122 is able to more easily pass into the sacrum, reducing operating time and patient discomfort.

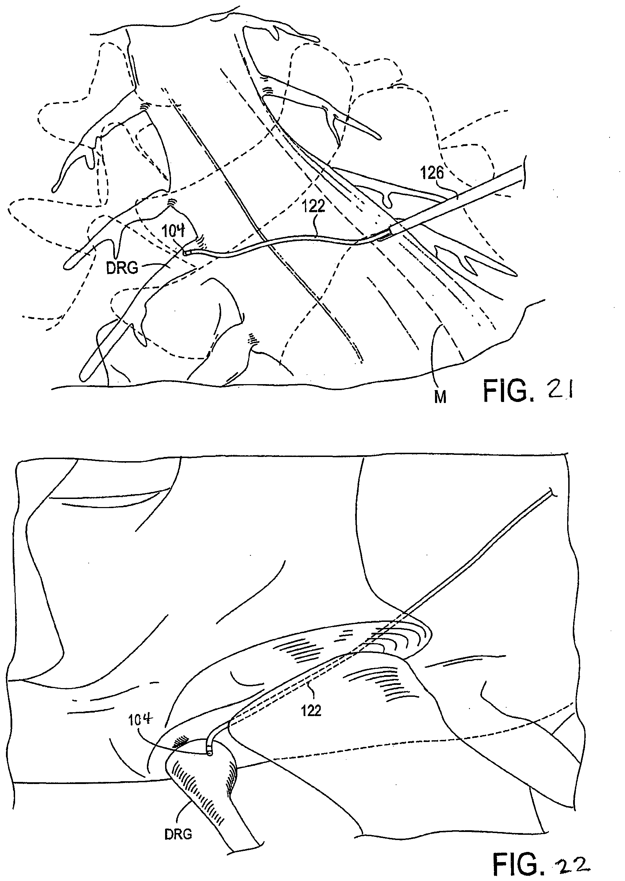

Referring to Fig. 21, the assembled sheath 122/lead 104/stylet 124 is advanced within the epidural space toward a DRG. Steering and manipulation is controlled proximally and is assisted by the construction of the assembled components and the precurvature of the sheath 122. In particular, the precurvature of the sheath 122 directs the lead 104 laterally outwardly, away from the midline M of the spinal column. Fig. 21 illustrates the assembled sheath 122/lead 104/stylet 124 advanced toward a DRG with the precurvature of the sheath 122 directing the lead 104 laterally outwardly.

Referring to Fig. 22, the lead 104/stylet 124 is then advanced beyond the distal end 128 of the sheath 122. In some cases, the lead 104 extends approximately 25.4 - 76.2 mm (1-3 inches) beyond the distal end 128 of the sheath 122. However, the lead 104 may extend any distance, such as less than 25.4 mm 6.35 - 76.2 mm (1 inch), (0.25-3 inches), or more than 76.2 mm (3 inches). Likewise, the sheath 122 may be retracted to expose the lead 104, with or without advancement of the lead 104. This may be useful when advancement of the lead 104 is restricted, such as by compression of the foraminal opening. The curvature of the stylet 124 within the lead 104 causes the lead 104 to bend further, along this curvature. This allows the laterally directed lead 104 to now curve around toward the DRG along the nerve root angulation. This two step curvature allows the lead 104 to be successfully steered to a desired position, such as having at least one of the electrodes 106 on, near or about the DRG. In addition, the ball shaped distal tip 118 resists trauma to the anatomy within the spinal column, such as the dural sac, ligaments and blood vessels, and resists imparting trauma to the DRG as the lead 104 is manipulated and advanced into place.

In some cases, the lead 104/stylet 124 is further advanced so that at least one of the electrodes 106 is positioned beyond the DRG. In some cases, the distal end of the lead 104/stylet 124 is extended through the foramen so as to position at least one electrode near the mixed spinal nerve SN, so as to selectively stimulate the mixed spinal nerve SN. In some cases, the lead 104/stylet 124 is advanced so that at least one electrode 106 is positioned along the medial branch MB of the dorsal ramus DRA. In other cases, the lead 104/stylet 124 is advanced further through the foramen so that at least one electrode 106 is positioned along a portion of the ventral ramus VRA. In fact, the lead 104/stylet 124 assembly may be advanced to any of the target anatomies described herein, wherein the stylet 124 assists in steering the lead 104. For example, when directing the lead 104 toward the medial branch MB, such movement is contrary to the natural advancement along the ventral ramus VRA. Thus, the curvature of the stylet 124 may be used to direct the lead 104 away from the ventral ramus VRA, along the medial branch MB. Other similar turns and contorsions through the anatomy to points along the peripheral nervous system may be navigated with the assistance of the internal stylet 124.

It may be appreciated that in some cases, the assembled sheath 122/lead 104/stylet 124 is advanced through the foramen to points therebeyond, such as along the peripheral nervous system. In such cases, the sheath 122 adds additional steering capabilities as described above. This may be of particular usefulness when reaching target anatomies at a greater distance from the foramen or at locations reachable through tortuous anatomy.

Once desirably positioned, the sheath 122 and stylet 124 are typically removed leaving the lead 104 in place. However, optionally, the stylet 124 may be left within the lead 104 to stabilize the lead 104, to assist in maintaining position and to resist migration. The target anatomy may then be stimulated by providing stimulation energy to the at least one electrode 106. It may be appreciated that multiple electrodes may be energized to stimulate the target anatomy or various anatomies at the same time. It may also be appreciated that the electrodes may be energized prior to removal of the stylet 124 and/or sheath 122, particularly to ascertain the desired positioning of the lead 104. It may further be appreciated that the sheath 122 may be retracted to expose the lead 104 rather than advancing the lead 104 therethrough.

Any number of leads 104 may be introduced through the same introducing needle 126. In some cases, the introducing needle 126 has more than one lumen, such as a double-barreled needle, to allow introduction of leads 100 through separate lumens. Further, any number of introducing needles 126 may be positioned along the spinal column for desired access to the epidural space. In some cases, a second needle is placed adjacent to a first needle. The second needle is used to deliver a second lead to a spinal level adjacent to the spinal level corresponding to the first needle. In some instances, there is a tract in the epidural space and the placement of a first lead may indicate that a second lead may be easily placed through the same tract. Thus, the second needle is placed so that the same epidural tract may be accessed. In other cases, a second needle is used to assist in stabilizing the tip of a sheath inserted through a first needle. In such cases, the second needle is positioned along the spinal column near the target anatomy. As the sheath is advanced, it may use the second needle to buttress against for stability or to assist in directing the sheath. This may be particularly useful when accessing a stenosed foramen which resists access. The proximal ends of the leads 104 are connected with an IPG which is typically implanted nearby.

### Examples Treatment Conditions

The devices, systems, and methods may be used to treat a variety of pain-related and non-pain related conditions. In particular, the devices, systems and methods may be used to treat the neurological diseases, disorders and stroke listed in Table 1. It may be appreciated that the present system may also be used to treat other diseases, disorders and conditions.

**Table 1. Neurological Diseases, Disorders and Stroke**

| |
|---|
| Acute Disseminated Encephalomyelitis |
| ADHD |
| Adie's Pupil |
| Adie's Syndrome |
| Adrenoleukodystrophy |
| Aqenesis of the Corpus Callosum |
| Agnosia |
| Aicardi Syndrome |
| Aicardi-Goutieres Syndrome Disorder |
| AIDS - Neurological Complications |
| Alexander Disease |
| Alpers' Disease |
| ALS (Amyotrophic Lateral Sclerosis) |
| Alternating Hemiplegia |
| Alzheimer's Disease |
| Anencephaly |
| Aneurysm |
| Angelman Syndrome |
| Angiomatosis |
| Anoxia |
| Antiphospholipid Syndrome |
| Aphasia |
| Apraxia |
| Arachnoid Cysts |
| Arachnoiditis |
| Arnold-Chiari Malformation |
| Arteriovenous Malformation |
| Asperger Syndrome |
| Ataxia |
| Ataxia Telangiectasia |
| Ataxias and Cerebellar or Spinocerebellar Degeneration |
| Atrial Fibrillation and Stroke |
| Attention Deficit-Hyperactivity Disorder |
| Autism |
| Autonomic Dysfunction |
| Back Pain |
| Barth Syndrome |
| Batten Disease |
| Becker's Myotonia |
| Behcet's Disease |
| Bell's Palsy |
| Benin Essential Blepharospasm |
| Benign Focal Amyotrophy |
| Benign Intracranial Hypertension |
| Bernhardt-Roth Syndrome |
| Binswanger's Disease |
| Blepharospasm |
| Bloch-Sulzberger Syndrome |
| Brachial Plexus Birth Injuries |
| Brachial Plexus Injuries |
| Bradbury-Eggleston Syndrome |
| Brain and Spinal Tumors |
| Brain Aneurysm |
| Brain Injury |
| Brown-Sequard Syndrome |
| Bulbospinal Muscular Atrophy |
| CADASIL |
| Canavan Disease |
| Carpal Tunnel Syndrome |
| Causalgia |
| Cavernomas |
| Cavernous Angioma |
| Cavernous Malformation |
| Central Cervical Cord Syndrome |
| Central Cord Syndrome |
| Central Pain Syndrome |
| Central Pontine Myelinolysis |
| Cephalic Disorders |
| Ceramidase Deficiency |
| Cerebellar Regeneration |
| Cerebellar Hypoplasia |
| Cerebral Aneurysm |
| Cerebral Arteriosclerosis |
| Cerebral Atrophy |
| Cerebral Beriberi |
| Cerebral Cavernous Malformation |
| Cerebral Giqantism |
| Cerebral Hypoxia |
| Cerebral Palsy |
| Cerebro-Oculo-Facio-Skeletal Syndrome (COFS) |
| Charcot-Marie-Tooth Disease |
| Chiari Malformation |
| Cholesterol Ester Storage Disease |
| Chorea |
| Choreoacanthocytosis |
| Chronic Inflammatory Demyelinating Polyneuropathy (CIDP) |
| Chronic Orthostatic Intolerance |
| Chronic Pain |
| Cockayne Syndrome Type II |
| Coffin Lowry Syndrome |
| Colpocephaly |
| Coma |
| Complex Regional Pain Syndrome |
| Congenital Facial Diplegia |
| Congenital Myasthenia |
| Congenital Myopathy |
| Congenital Vascular Cavernous Malformations |
| Corticobasal Degeneration |
| Cranial Arteritis |
| Craniosynostosis |
| Cree encephalitis |
| Creutzfeldt-Jakob Disease |
| Cumulative Trauma Disorders |
| Cushing's Syndrome |
| Cytomegalic Inclusion Body Disease |
| Cytomegalovirus Infection |
| Dancing Eyes-Dancing Feet Syndrome |
| Dandy-Walker Syndrome |
| Dawson Disease |
| De Morsier's Syndrome |
| Dejerine-Klumpke Palsy |
| Dementia |
| Dementia - Multi-Infarct |
| Dementia - Semantic |
| Dementia - Subcortical |
| Dementia With Lewy Bodies |
| Dentate Cerebellar Ataxia |
| Dentatorubral Atrophy |
| Dermatomyositis |
| Developmental Dyspraxia |
| Devic's Syndrome |
| Diabetic Neuropathy |
| Diffuse Sclerosis |
| Dravet Syndrome |
| Dysautonomia |
| Dysgraphia |
| Dyslexia |
| Dysphagia |
| Dyspraxia |
| Dyssynergia Cerebellaris Myoclonica |
| Dyssynergia Cerebellaris Progressiva |
| Dystonias |
| Early Infantile Epileptic Encephalopathy |
| Empty Sella Syndrome |
| Encephalitis |
| Encephalitis Lethargica |
| Encephaloceles |
| Encephalopathy |
| Encephalopathy (familial infantile) |
| Encephalotrigeminal Angiomatosis |
| Epilepsy |
| Epileptic Hemiplegia |
| Erb-Duchenne and Dejerine-Klumpke Palsies |
| Erb's Palsy |
| Essential Tremor |
| Extrapontine Myelinolysis |
| Fabry Disease |
| Fahr's Syndrome |
| Fainting |
| Familial Dysautonomia |
| Familial Hemangioma |
| Familial Idiopathic Basal Ganglia Calcification |
| Familial Periodic Paralyses |
| Familial Spastic Paralysis |
| Farber's Disease |
| Febrile Seizures |
| Fibromuscular Dysplasia |
| Fisher Syndrome |
| Floppy Infant Syndrome |
| Foot Drop |
| Friedreich's Ataxia |
| Frontotemporal Dementia |
| Gangliosidoses |
| Gaucher's Disease |
| Gerstmann's Syndrome |
| Gerstmann-Straussler-Scheinker Disease |
| Giant Axonal Neuropathy |
| Giant Cell Arteritis |
| Giant Cell Inclusion Disease |
| Globoid Cell Leukodystrophy |
| Glossopharyngeal Neuralgia |
| Glycogen Storage Disease |
| Guillain-Barré Syndrome |
| Hallervorden-Spatz Disease |
| Head Injury |
| Headache |
| Hemicrania Continua |
| Hemifacial Spasm |
| Hemiplegia Alterans |
| Hereditary Neuropathies |
| Hereditary Spastic Paraplegia |
| Heredopathia Atactica Polyneuritiformis |
| Herpes Zoster |
| Herpes Zoster Oticus |
| Hirayama Syndrome |
| Holmes-Adie syndrome |
| Holoprosencephaly |
| HTLV-1 Associated Myelopathy |
| Huqhes Syndrome |
| Huntington's Disease |
| Hydranencephaly |
| Hydrocephalus |
| Hydrocephalus - Normal Pressure |
| Hydromyelia |
| Hypercortisolism |
| Hypersomnia |
| Hypertonia |
| Hypotonia |
| Hypoxia |
| Immune-Mediated Encephalomyelitis |
| Inclusion Body Myositis |
| Incontinentia Pigmenti |
| Infantile Hypotonia |
| Infantile Neuroaxonal Dystrophy |
| Infantile Phytanic Acid Storage Disease |
| Infantile Refsum Disease |
| Infantile Spasms |
| Inflammatory Myopathies |
| Iniencephaly |
| Intestinal Lipodystrophy |
| Intracranial Cysts |
| Intracranial Hypertension |
| Isaac's Syndrome |
| Joubert Syndrome |
| Kearns-Sayre Syndrome |
| Kennedy's Disease |
| Kinsbourne syndrome |
| Kleine-Levin Syndrome |
| Klippel-Feil Syndrome |
| Klippel-Trenaunay Syndrome (KTS) |
| Klüver-Bucy Syndrome |
| Korsakoff's Amnesic Syndrome |
| Krabbe Disease |
| Kugelberg-Welander Disease |
| Kuru |
| Lambert-Eaton Myasthenic Syndrome |
| Landau-Kleffner Syndrome |
| Lateral Femoral Cutaneous Nerve Entrapment |
| Lateral Medullary Syndrome |
| Learning Disabilities |
| Leigh's Disease |
| Lennox-Gastaut Syndrome |
| Lesch-Nyhan Syndrome |
| Leukodystrophy |
| Levine-Critchley Syndrome |
| Lewy Body Dementia |
| Lipid Storage Diseases |
| Lipoid Proteinosis |
| Lissencephaly |
| Locked-In Syndrome |
| Lou Gehrig's Disease |
| Lupus - Neurological Sequelae |
| Lyme Disease - Neurological Complications |
| Machado-Joseph Disease |
| Macrencephaly |
| Megalencephaly |
| Melkersson-Rosenthal Syndrome |
| Meningitis |
| Meningitis and Encephalitis |
| Menkes Disease |
| Meralgia Paresthetica |
| Metachromatic Leukodystrophy |
| Microcephaly |
| Migraine |
| Miller Fisher Syndrome |
| Mini Stroke |
| Mitochondrial Myopathies |
| Moebius Syndrome |
| Monomelic Amyotrophy |
| Motor Neuron Diseases |
| Moyamoya Disease |
| Mucolipidoses |
| Mucopolysaccharidoses |
| Multifocal Motor Neuropathy |
| Multi-Infarct Dementia |
| Multiple Sclerosis |
| Multiple System Atrophy |
| Multiple System Atrophy with Orthostatic Hypotension |
| Muscular Dystrophy |
| Myasthenia - Congenital |
| Myasthenia Gravis |
| Myelinoclastic Diffuse Sclerosis |
| Myoclonic Encephalopathy of Infants |
| Myoclonus |
| Myopathy |
| Myopathy - Congenital |
| Myopathy - Thyrotoxic |
| Myotonia |
| Myotonia Congenita |
| Narcolepsy |
| Neuroacanthocytosis |
| Neurodegeneration with Brain Iron Accumulation |
| Neurofibromatosis |
| Neuroleptic Malignant Syndrome |
| Neurological Complications of AIDS |
| Neurological Complications of Lyme Disease |
| Neurological Consequences of Cytomegalovirus Infection |
| Neurological Manifestations of Pompe Disease |
| Neurological Sequelae Of Lupus |
| Neuromyelitis Optica |
| Neuromyotonia |
| Neuronal Ceroid Lipofuscinosis |
| Neuronal Migration Disorders |
| Neuropathy - Hereditary |
| Neurosarcoidosis |
| Neurosyphilis |
| Neurotoxicity |
| Nevus Cavernosus |
| Niemann-Pick Disease |
| Normal Pressure Hydrocephalus |
| Occipital Neuralgia |
| Ohtahara Syndrome |
| Olivopontocerebellar Atrophy |
| Opsoclonus Myoclonus |
| Orthostatic Hypotension |
| O'Sullivan-McLeod Syndrome |
| Overuse Syndrome |
| Pain |
| Pantothenate Kinase-Associated Neurodegeneration |
| Paraneoplastic Syndromes |
| Parkinson's Disease |
| Paroxysmal Choreoathetosis |
| Paroxysmal Hemicrania |
| Parry-Rom berg |
| Pelizaeus-Merzbacher Disease |
| Pena Shokeir II Syndrome |
| Perineural Cysts |
| Periodic Paralyses |
| Peripheral Neuropathy |
| Periventricular Leukomalacia |
| Persistent Vegetative State |
| Pervasive Developmental Disorders |
| Phytanic Acid Storage Disease |
| Pick's Disease |
| Pinched Nerve |
| Piriformis Syndrome |
| Pituitary Tumors |
| Polymyositis |
| Pompe Disease |
| Porencephaly |
| Postherpetic Neuralgia |
| Postinfectious Encephalomyelitis |
| Post-Polio Syndrome |
| Postural Hypotension |
| Postural Orthostatic Tachycardia Syndrome |
| Postural Tachycardia Syndrome |
| Primary Dentatum Atrophy |
| Primary Lateral Sclerosis |
| Primary Progressive Aphasia |
| Prion Diseases |
| Progressive Hemifacial Atrophy |
| Progressive Locomotor Ataxia |
| Progressive Multifocal Leukoencephalopathy |
| Progressive Sclerosing Poliodystrophy |
| Progressive Supranuclear Palsy |
| Prosopagnosia |
| Pseudo-Torch syndrome |
| Pseudotoxoplasmosis syndrome |
| Pseudotumor Cerebri |
| Ramsay Hunt Syndrome I |
| Ramsay Hunt Syndrome II |
| Rasmussen's Encephalitis |
| Reflex Sympathetic Dystrophy Syndrome |
| Refsum Disease |
| Refsum Disease - Infantile |
| Repetitive Motion Disorders |
| Repetitive Stress Injuries |
| Restless Legs Syndrome |
| Retrovirus-Associated Myelopathy |
| Rett Syndrome |
| Reye's Syndrome |
| Rheumatic Encephalitis |
| Riley-Day Syndrome |
| Sacral Nerve Root Cysts |
| Saint Vitus Dance |
| Salivary Gland Disease |
| Sandhoff Disease |
| Schilder's Disease |
| Schizencephaly |
| Seitelberger Disease |
| Seizure Disorder |
| Semantic Dementia |
| Septo-Optic Dysplasia |
| Severe Myoclonic Epilepsy of Infancy (SMEI) |
| Shaken Baby Syndrome |
| Shingles |
| Shy-Drager Syndrome |
| Sjögren's Syndrome |
| Sleep Apnea |
| Sleeping Sickness |
| Sotos Syndrome |
| Spasticity |
| Spina Bifida |
| Spinal Cord Infarction |
| Spinal Cord Injury |
| Spinal Cord Tumors |
| Spinal Muscular Atrophy |
| Spinocerebellar Atrophy |
| Spinocerebellar Degeneration |
| Steele-Richardson-Olszewski Syndrome |
| Stiff-Person Syndrome |
| Striatonigral Degeneration |
| Stroke |
| Sturge-Weber Syndrome |
| Subacute Sclerosing Panencephalitis |
| Subcortical Arteriosclerotic Encephalopathy |
| SUNCT Headache |
| Swallowing Disorders |
| Sydenham Chorea |
| Syncope |
| Syphilitic Spinal Sclerosis |
| Syringohydromyelia |
| Syringomyelia |
| Systemic Lupus Erythematosus |
| Tabes Dorsalis |
| Tardive Dyskinesia |
| Tarlov Cysts |
| Tay-Sachs Disease |
| Temporal Arteritis |
| Tethered Spinal Cord Syndrome |
| Thomsen's Myotonia |
| Thoracic Outlet Syndrome |
| Thyrotoxic Myopathy |
| Tic Douloureux |
| Todd's Paralysis |
| Tourette Syndrome |
| Transient Ischemic Attack |
| Transmissible Spongiform Encephalopathies |
| Transverse Myelitis |
| Traumatic Brain Injury |
| Tremor |
| Trigeminal Neuralgia |
| Tropical Spastic Paraparesis |
| Troyer Syndrome |
| Tuberous Sclerosis |
| Vascular Erectile Tumor |
| Vasculitis Syndromes of the Central and Peripheral Nervous Systems |
| Von Economo's Disease |
| Von Hippel-Lindau Disease (VHL) |
| Von Recklinghausen's Disease |
| Wallenberg's Syndrome |
| Werdnig-Hoffman Disease |
| Wernicke-Korsakoff Syndrome |
| West Syndrome |
| Whiplash |
| Whipple's Disease |
| Williams Syndrome |
| Wilson's Disease |
| Wolman's Disease |
| X-Linked Spinal and Bulbar Muscular Atrophy |
| Zellweger Syndrome |

It may also be appreciated that although the methods, systems and devices have been described and illustrated herein as using an epidural approach, other approaches may be used including extra-forminal wherein the DRG or various nerves are approached from outside of the spinal column, not crossing the midline of the spinal cord and/or not entering the epidural space. It may also be appreciated that the DRG or various nerves may be approached through a pedicle. For example, a needle may be advanced through the pedicle and the lead delivered through the needle.

It may be appreciated that in some cases, the systems or devices deliver an agent or drug formulation to one or more target spinal anatomies, e.g., a dorsal root ganglion, mixed spinal nerve SN, ventral ramus VRA, dorsal ramus DRA, lateral branch LB, medial branch MB, intermediate branch IB, and/or rami communicantes (white ramus and/or gray ramus), and/or portions of the peripheral nervous system, to name a few. It may be appreciated that in some cases, the device, method and system is configured to enable direct and specific electrical stimulation of the target anatomy in combination with delivery of the agent. For example, in some cases, electrical stimulation is in a temporal pattern which is coordinated with a temporal pattern of delivery of the agent. In some embodiments, combined delivery of electrical stimulation and agent delivery results in one or more effects, including but not limited to, (i) synergistic action of the agent and electrical stimulation, (ii) an increase in the selectivity of an agent to target DRG cell bodies, (iii) targeted activation of an agent delivered to the DRG and (iv) differential enhancement of an agent to delivered target DRG cell bodies.

In many of the cases described above, the electronic circuitry of an IPG (e.g., 102) and the components of a programmer (e.g., 122) can be considered a data processing system of a stimulation system. Generally, the data processing system included may include at least one processor (or other controller), which will typically include circuitry implanted in the patient, circuitry external of the patient, or both. When external processor circuitry is included in the data processing system, it may include one or more proprietary processor boards and/or may make use of a general purpose desktop computer, notebook computer handheld computer. The external processor may communicate with a number of peripheral devices (and/or other processors) via a bus subsystem, and these peripheral devices may include a data and/or programming storage subsystem or memory. The peripheral devices may also include one or more user interface input devices, user interface output devices and a network interface subsystem to provide an interface with other processing systems and networks such as the Internet, an intranet and/or an Ethernet™. Implanted circuitry of the processor system may have some or all of the constituent components described above for external circuitry, with peripheral devices that provide user input, user output and networking generally employing wireless communication capabilities, although hard-wired solutions or other trans-cutaneous telemetry techniques could also be employed.

An external or implanted memory of the processor system can be used to store, in a tangible storage media, machine readable instructions or programming in the form of a computer executable code embodying one or more of the methods described herein. The memory may also similarly store data for implementing one or more of these methods. The memory may, for example, include a random access memory (RAM) for storage of instructions and data during program execution and/or a read only memory (ROM) in which fixed instructions are stored. Persistent (non-volatile) storage may be provided and/or the memory may include a hard disk drive, a compact digital read only memory (CD-ROM) drive, an optical drive, DVD, CD-R, CD-RW, solid-state removable memory, and/or other fixed or removable media cartridges or disks. Some or all of the stored programming code may be altered after implantation and/or initial use of the device to alter functionality of the stimulator system. More generally, a computer readable medium can be provided including instructions stored thereon which when read and executed by one or more processors cause the one or more processors to perform various steps described above.

The invention is defined by the claims.

## Claims

1. A system (100) configured for treating pain in a patient, the system comprising:
an implantable neurostimulator (102) including a controller (109), memory (149) and telemetry circuitry (148);
an implantable lead (104) connected to the neurostimulator, the lead (104) having at least one electrode (106) wherein at least one of the at least one electrodes (106) is configured to be positioned near a dorsal root ganglion associated with the pain; and
a non-implantable programmer (122) including a controller (144), memory (149) and telemetry circuitry (148),
wherein the programmer (122) is configured to select at least one stimulation parameter for providing energy to the at least one of the at least one electrodes (106) for providing selective stimulation of at least a portion of the dorsal root ganglion, wherein the selective stimulation is for providing a massaging sensation for treating the pain, and
wherein the telemetry circuitry (148) of the programmer is configured to send instructions to the neurostimulator (102) for providing the energy to the at least one of the at least one electrodes (106) and for providing the massaging sensation for treating the pain,
**characterized in that** the system comprises a sensor configured to detect muscle contractions and positionable to detect contractions of a muscle innervated by motor nerves exiting close to the dorsal root ganglion and **in that** the programmer is configured to adjust a stimulation frequency from a frequency above 10 Hz to a frequency at which a muscle contraction is first detected by the sensor and to then increase the stimulation frequency to a frequency of 16-20 Hz to transition to treatment.

2. A system (100) as in claim 1, wherein the lead (104) further comprises at least one further electrode (106), wherein at least one of the at least one further electrodes (106) is configured to be positioned near a mixed spinal nerve, a dorsal ramus, a lateral branch, a medial branch, an intermediate branch and/or a ventral ramus associated with the dorsal root ganglion while the at least one of the at least one electrodes (106) is positioned near the dorsal root ganglion.

3. A system (100) as in claim 2, wherein the programmer (122) is also configured to select at least one stimulation parameter for providing energy to the at least one of the at least one further electrode (106) for providing selective stimulation of the mixed spinal nerve, the dorsal ramus, the lateral branch, the medial branch, the intermediate branch and/or the ventral ramus.

4. A system (100) as in claims 1 or 3, wherein the at least one stimulation parameter comprises selection of the at least one of the at least one electrode (106) and/or the at least one of the further electrode (106) to receive stimulation, and/or
wherein the at least one stimulation parameter comprises frequency, amplitude or pulse width, preferably
wherein the at least one stimulation parameter comprises a frequency of at least 16 Hz, preferably
wherein the at least one stimulation parameter comprises a frequency in the range of approximately 20-50 Hz, preferably
wherein the at least one stimulation parameter comprises a frequency of approximately 20 Hz.

5. A system (100) as in claim 1, wherein the programmer (122) is further configured to select at least one stimulation parameter for providing energy to the at least one of the at least one electrodes (106) for resulting in an undesired muscle contraction, wherein the undesired muscle contraction is indicative of the at least one of the at least one electrodes (106) being positioned near the dorsal root ganglion associated with the pain.

6. A system (100) as in claim 5, wherein the at least one stimulation parameter used to provide energy to the at least one of the at least one electrodes (106) for resulting in undesired muscle contractions has a frequency of less than or equal to approximately 10Hz, and/or
the system further comprises a sensor configured to detect the resultant undesired muscle contraction, and/or
the programmer (122) is configured to enable an increase in the frequency to at least 16Hz to transition the undesired muscle contractions to the massaging sensation for treating the pain.

7. A system (100) as in claim 1, wherein:
the at least one electrode (106) comprises a plurality of electrodes (106);
the at least one stimulation parameter comprises a combination of the electrodes (106);
the non-implanted programmer (122) is configured to cause testing, by the implantable neurostimulator (102), of a plurality of different combinations of the electrodes (106) to identify which combination of the electrodes (106) results in undesired muscle contractions when used to selectively stimulate at least a portion of the dorsal root ganglion at a first frequency; and
the non-implanted programmer (122) is configured to increase the first frequency to a second frequency to transition the undesired muscle contractions to the massaging sensation which treats the pain, after the combination of the electrodes (106) which results in the undesired muscle contractions is identified.

8. A system (100) as in claim 7, wherein:
the first frequency is less than or equal to approximately 10Hz; and
the second frequency is at least 16Hz, and/or
the system further comprises a sensor configured to detect the undesired muscle contractions.

9. A system as in claim 7, the system comprising:
means for causing testing of a plurality of different stimulation parameters that are used for selective stimulation of at least a portion of a dorsal root ganglion associated with pain in a patient that is to be treated;
means for identifying when the selective stimulation causes a massaging sensation for treating the pain; and
means for saving information corresponding to at least one stimulation parameter that causes the massaging sensation which treats the pain.

10. The system of claim 9, wherein the means for identifying includes means for receiving an indication that the selective stimulation causes a massaging sensation for treating the pain.

11. The system of claim 9, wherein the means for identifying includes a means for sensing when selective stimulation, having a frequency of less than or equal to 10Hz, causes undesired muscle contractions indicative of at least one electrode being used to deliver the selective stimulation being positioned near the dorsal root ganglion associated with the pain, preferably
wherein the means for identifying further includes a means for increasing the frequency to at least 16Hz to transition the undesired muscle contractions to the massaging sensation for treating the pain.

12. A computer readable medium, including instructions stored thereon which when read and executed by one or more processors cause the one or more processors to perform the steps comprising:
testing of a plurality of different stimulation parameters that are used for selective stimulation of at least a portion of a dorsal root ganglion associated with pain in a patient that is to be treated;
identifying when the selective stimulation causes a massaging sensation for treating the pain; and
saving information corresponding to at least one stimulation parameter that causes the massaging sensation for treating the pain, **characterized in that**
the steps further comprising adjusting a stimulation frequency from a frequency above 10 Hz to a frequency at which an indication is first received from a sensor that a muscle contraction is detected and then increasing the stimulation frequency to a frequency of 16-20 Hz to transition to treatment.

13. The computer readable medium of claim 12, wherein the identifying includes receiving an indication that the selective stimulation causes a massaging sensation which treats the pain.

14. The computer readable medium of claim 12, further comprising identifying when selective stimulation causes an undesired muscle contraction indicative of at least one electrode being used to deliver the selective stimulation being positioned near the dorsal root ganglion associated with the pain, preferably
wherein the identifying when selective stimulation causes an undesired muscle contraction comprises sensing when selective stimulation causes an undesired muscle contraction.

15. The computer readable medium claim 12, wherein the identifying further comprises increasing a frequency of less than or equal to 10Hz to a frequency of least 16Hz to transition the undesired muscle contraction to the massaging sensation for treating the pain.

## Patentansprüche

1. System (100), das zur Schmerzbehandlung eines Patienten konfiguriert ist, wobei das System umfasst:
einen implantierbaren Neurostimulator (102) mit einer Steuereinheit (109), einem Speicher (149) und einer Telemetrieschaltungsanordnung (148);
eine implantierbare Leitung (104), die mit dem Neurostimulator verbunden ist, wobei die Leitung (104) eine oder mehr Elektroden (106) hat, wobei eine oder mehr der einen oder mehr Elektroden (106) zur Positionierung in der Nähe eines mit dem Schmerz assoziierten Hinterwurzelganglions konfiguriert sind; und
eine nichtimplantierbare Programmiereinheit (122) mit einer Steuereinheit (144), einem Speicher (149) und einer Telemetrieschaltungsanordnung (148),
wobei die Programmiereinheit (122) zum Auswählen von wenigstens einem Stimulationsparameter zur Versorgung der einen oder mehr der einen oder mehr Elektroden (106) mit Energie zur Bereitstellung selektiver Stimulation wenigstens eines Teils des Hinterwurzelganglions konfiguriert ist, wobei die selektive Stimulation zum Bereitstellen eines Massagegefühls zur Behandlung des Schmerzes ist, und
wobei die Telemetrieschaltungsanordnung (148) der Programmiereinheit zum Senden von Anweisungen an den Neurostimulator (102) zur Versorgung der einen oder mehr der einen oder mehr Elektroden (106) mit der Energie und zur Bereitstellung des Massagegefühls zur Behandlung des Schmerzes konfiguriert ist, **dadurch gekennzeichnet, dass** das System einen Sensor aufweist, der zum Erkennen von Muskelkontraktionen konfiguriert ist und zum Erkennen von Kontraktionen eines Muskels positionierbar ist, der von motorischen Nerven innerviert wird, die in der Nähe des Hinterwurzelganglions austreten, und dadurch, dass die Programmiereinheit zum Einstellen einer Stimulationsfrequenz von einer Frequenz über 10 Hz auf eine Frequenz, bei der eine Muskelkontraktion von dem Sensor zuerst erkannt wird, und dann zum Erhöhen der Frequenz auf eine Frequenz von 16 bis 20 Hz, um zur Behandlung überzugehen, konfiguriert ist.

2. System (100) nach Anspruch 1, wobei die Leitung (104) ferner eine oder mehr weitere Elektroden (106) aufweist, wobei eine oder mehr der einen oder mehr weiteren Elektroden (106) konfiguriert sind, um in der Nähe eines gemischten Spinalnervs, eines dorsalen Ramus, eines lateralen Astes, eines medialen Astes, eines intermediären Astes und/oder eines ventralen Ramus, der dem Hinterwurzelganglion zugeordnet ist, positioniert zu werden, während die eine oder mehr der einen oder mehr Elektroden (106) in der Nähe des Hinterwurzelganglions positioniert wird.

3. System (100) nach Anspruch 2, wobei die Programmiereinheit (122) auch zum Auswählen von wenigstens einem Stimulationsparameter zur Versorgung der einen oder mehr der einen oder mehr weiteren Elektroden (106) mit Energie zur Bereitstellung selektiver Stimulation des gemischten Spinalnervs, des dorsalen Ramus, des lateralen Astes, des medialen Astes, des intermediären Astes und/oder des ventralen Ramus konfiguriert ist.

4. System (100) nach Anspruch 1 oder 3, wobei der wenigstens eine Stimulationsparameter eine Auswahl der einen oder mehr der einen oder mehr Elektroden (106) und/oder der einen oder mehr der weiteren Elektroden (106) zum Erhalten der Stimulation aufweist und/oder
wobei der wenigstens eine Stimulationsparameter Frequenz, Amplitude oder Pulsdauer umfasst, vorzugsweise
wobei der wenigstens eine Stimulationsparameter eine Frequenz von wenigstens 16 Hz umfasst, vorzugsweise
wobei der wenigstens eine Stimulationsparameter eine Frequenz im Bereich von etwa 20 bis 50 Hz umfasst, vorzugsweise
wobei der wenigstens eine Stimulationsparameter eine Frequenz von etwa 20 Hz umfasst.

5. System (100) nach Anspruch 1, wobei die Programmiereinheit (122) ferner zum Auswählen von wenigstens einem Stimulationsparameter zur Versorgung der einen oder mehr der einen oder mehr Elektroden (106) mit Energie, um eine unerwünschte Muskelkontraktion zu bewirken, wobei die unerwünschte Muskelkontraktion darauf schließen lässt, dass die eine oder mehr der einen oder mehr Elektroden (106) in der Nähe des mit dem Schmerz assoziierten Hinterwurzelganglions positioniert sind.

6. System (100) nach Anspruch 5, wobei der wenigstens eine Stimulationsparameter, der zur Versorgung der einen oder mehr der einen oder mehr Elektroden (106) mit Energie, um unerwünschte Muskelkontraktionen zu bewirken, verwendet wird, eine Frequenz von kleiner oder gleich etwa 10 Hz hat und/oder
das System ferner einen Sensor aufweist, der zum Erkennen der bewirkten unerwünschten Muskelkontraktion konfiguriert ist, und/oder
die Programmiereinheit (122) zum Ermöglichen einer Erhöhung der Frequenz auf wenigstens 16 Hz konfiguriert ist, um die unerwünschten Muskelkontraktionen in das Massagegefühl zur Behandlung des Schmerzes übergehen zu lassen.

7. System (100) nach Anspruch 1, wobei:
die eine oder mehr Elektroden (106) mehrere Elektroden (106) umfassen;
der wenigstens eine Stimulationsparameter eine Kombination der Elektroden (106) umfasst;
die nichtimplantierte Programmiereinheit (122) konfiguriert ist, um Testen von mehreren verschiedenen Kombinationen der Elektroden (106) durch den implantierbaren Neurostimulator (102) zu veranlassen, um festzustellen, welche Kombination der Elektroden (106) bei Verwendung zum selektiven Stimulieren von wenigstens eines Teils des Hinterwurzelganglions mit einer ersten Frequenz unerwünschte Muskelkontraktionen bewirkt; und
die nichtimplantierte Programmiereinheit (122) zum Erhöhen der ersten Frequenz auf eine zweite Frequenz konfiguriert ist, um die unerwünschten Muskelkontraktionen in das Massagegefühl, das den Schmerz behandelt, übergehen zu lassen, nachdem die Kombination der Elektroden (106), welche die unerwünschten Muskelkontraktionen bewirkt, festgestellt worden ist.

8. System (100) nach Anspruch 7, wobei:
die erste Frequenz kleiner oder gleich etwa 10 Hz ist; und
die zweite Frequenz wenigstens 16 Hz beträgt und/oder
das System ferner einen Sensor aufweist, der zum Erkennen der unerwünschten Muskelkontraktionen konfiguriert ist.

9. System nach Anspruch 7, wobei das System aufweist:
ein Mittel zum Veranlassen des Testens von mehreren verschiedenen Stimulationsparametern, die zur selektiven Stimulation wenigstens eines mit zu behandelndem Schmerz eines Patienten assoziierten Teils eines Hinterwurzelganglions konfiguriert ist;
ein Mittel zum Erkennen, wann die selektive Stimulation ein Massagegefühl zur Behandlung des Schmerzes hervorruft; und
ein Mittel zum Speichern von Informationen, die wenigstens einem Stimulationsparameter entsprechen, der das Massagegefühl hervorruft, das den Schmerz behandelt.

10. System nach Anspruch 9, wobei das Mittel zum Erkennen ein Mittel zum Empfangen eines Hinweises, dass die selektive Stimulation ein Massagegefühl zur Behandlung des Schmerzes hervorruft, enthält.

11. System nach Anspruch 9, wobei das Mittel zum Erkennen ein Mittel zum Erfassen enthält, wann selektive Stimulation mit einer Frequenz von kleiner oder gleich 10 Hz unerwünschte Muskelkontraktionen veranlasst, die darauf schließen lassen, dass eine oder mehr zur Abgabe der selektiven Stimulation verwendete Elektroden in der Nähe des mit dem Schmerz assoziierten Hinterwurzelganglions positioniert sind, vorzugsweise
wobei das Mittel zum Erkennen ferner ein Mittel zum Erhöhen der Frequenz auf wenigstens 16 Hz enthält, um die unerwünschten Muskelkontraktionen in das Massagegefühl zur Behandlung des Schmerzes übergehen zu lassen.

12. Computerlesbares Medium, das darauf gespeicherte Anweisungen enthält, die bei Lesen und Ausführung durch einen oder mehr Prozessoren die einen oder mehr Prozessoren zum Durchführen der Schritte veranlasst, die Folgendes umfassen:
Testen von mehreren verschiedenen Stimulationsparametern, die zur selektiven Stimulation wenigstens eines mit zu behandelndem Schmerz eines Patienten assoziierten Teils eines Hinterwurzelganglions konfiguriert ist;
Erkennen, wann die selektive Stimulation ein Massagegefühl zur Behandlung des Schmerzes hervorruft; und
Speichern von Informationen, die wenigstens einem Stimulationsparameter entsprechen, der das Massagegefühl zur Behandlung des Schmerzes hervorruft, **dadurch gekennzeichnet, dass**
die Schritte ferner das Einstellen einer Stimulationsfrequenz von einer Frequenz über 10 Hz auf eine Frequenz, bei der ein Hinweis, dass eine Muskelkontraktion erkannt wird, zuerst von einem Sensor empfangen wird, und dann Erhöhen der Stimulationsfrequenz auf eine Frequenz von 16 bis 20 Hz, um zur Behandlung überzugehen, enthalten.

13. Computerlesbares Medium nach Anspruch 12, wobei das Erkennen das Empfangen eines Hinweises enthält, dass die selektive Stimulation ein Massagegefühl hervorruft, das den Schmerz behandelt.

14. Computerlesbares Medium nach Anspruch 12, das ferner das Erkennen aufweist, wann selektive Stimulation eine unerwünschte Muskelkontraktion veranlasst, die darauf schließen lässt, dass eine oder mehr zur Abgabe der selektiven Stimulation verwendete Elektroden in der Nähe des mit dem Schmerz assoziierten Hinterwurzelganglions positioniert sind, vorzugsweise
wobei das Erkennen, wann selektive Stimulation eine unerwünschte Muskelkontraktion veranlasst, das Erfassen enthält, wann selektive Stimulation eine unerwünschte Muskelkontraktion veranlasst.

15. Computerlesbares Medium nach Anspruch 14, wobei das Erkennen ferner das Erhöhen einer Frequenz von kleiner oder gleich 10 Hz auf eine Frequenz von wenigstens 16 Hz enthält, um die unerwünschte Muskelkontraktion in das Massagegefühl zur Behandlung des Schmerzes übergehen zu lassen.

## Revendications

1. Système (100) configuré pour traiter la douleur chez un patient, le système comprenant :
un neurostimulateur implantable (102) comprenant un contrôleur (109), une mémoire (149) et un circuit de télémétrie (148) ;
un câble implantable (104) connecté au neurostimulateur, le câble (104) comportant au moins une électrode (106), au moins l'une desdites au moins une électrode (106) étant configurée pour être positionnée près d'un ganglion rachidien associé à la douleur ; et
un programmateur non implantable (122) comprenant un contrôleur (144), une mémoire (149) et un circuit de télémétrie (148),
dans lequel le programmateur (122) est configuré pour sélectionner au moins un paramètre de stimulation pour apporter de l'énergie à ladite au moins l'une desdites au moins une électrode (106) pour produire une stimulation sélective d'au moins une partie du ganglion rachidien, la stimulation sélective servant à produire une sensation de massage pour traiter la douleur, et
dans lequel le circuit de télémétrie (148) du programmateur est configuré pour envoyer des instructions au neurostimulateur (102) pour apporter l'énergie à ladite au moins l'une desdites au moins une électrode (106) et pour produire la sensation de massage pour le traitement de la douleur, **caractérisé en ce que** le système comprend un capteur configuré pour détecter les contractions musculaires et pouvant être positionné pour détecter les contractions d'un muscle innervé par des nerfs moteurs sortant près du ganglion rachidien et **en ce que** le programmateur est configuré pour ajuster une fréquence de stimulation, d'une fréquence supérieure à 10 Hz à une fréquence à laquelle une contraction musculaire est d'abord détectée par le capteur, en augmentant ensuite la fréquence de stimulation jusqu'à une fréquence de 16 à 20 Hz pour passer au traitement.

2. Système (100) selon la revendication 1, dans lequel le câble (104) comprend en outre au moins une électrode supplémentaire (106), dans lequel au moins l'une desdites au moins une électrode supplémentaire (106) est configurée pour être positionnée près d'un nerf spinal mixte, d'un rameau dorsal, d'une branche latérale, d'une branche médiale, d'une branche intermédiaire et/ou d'un rameau ventral associé(e) au ganglion rachidien tandis que ladite au moins l'une desdites au moins une électrode (106) est positionnée près du ganglion rachidien.

3. Système (100) selon la revendication 2, dans lequel le programmateur (122) est également configuré pour sélectionner au moins un paramètre de stimulation pour apporter de l'énergie à ladite au moins l'une desdites au moins une électrode supplémentaire (106) pour produire une stimulation sélective du nerf spinal mixte, du rameau dorsal, de la branche latérale, de la branche médiale, de la branche intermédiaire et/ou du rameau ventral.

4. Système (100) selon les revendications 1 ou 3, dans lequel ledit au moins un paramètre de stimulation comprend la sélection de ladite au moins l'une desdites au moins une électrode (106) et/ou de ladite au moins l'une des électrodes supplémentaires (106) pour recevoir la stimulation, et/ou
dans lequel ledit au moins un paramètre de stimulation comprend la fréquence, l'amplitude ou la largeur d'impulsion, préférablement
dans lequel ledit au moins un paramètre de stimulation comprend une fréquence d'au moins 16 Hz, préférablement
dans lequel ledit au moins un paramètre de stimulation comprend une fréquence d'environ 20 à 50 Hz, préférablement
dans lequel ledit au moins un paramètre de stimulation comprend une fréquence d'environ 20 Hz.

5. Système (100) selon la revendication 1, dans lequel le programmateur (122) est en outre configuré pour sélectionner au moins un paramètre de stimulation pour apporter de l'énergie à ladite au moins l'une desdites au moins une électrode (106) pour entraîner une contraction musculaire indésirable, la contraction musculaire indésirable indiquant que ladite au moins l'une desdites au moins une électrode (106) est positionnée près du ganglion rachidien associé à la douleur.

6. Système (100) selon la revendication 5, dans lequel ledit au moins un paramètre de stimulation utilisé pour apporter de l'énergie à ladite au moins l'une desdites au moins une électrode (106) pour produire des contractions musculaires indésirables a une fréquence inférieure ou égale à environ 10 Hz, et/ou
le système comprend en outre un capteur configuré pour détecter la contraction musculaire indésirable ainsi produite, et/ou
le programmateur (122) est configuré pour permettre une augmentation de la fréquence jusqu'à au moins 16 Hz pour passer des contractions musculaires indésirables à la sensation de massage pour le traitement de la douleur.

7. Système (100) selon la revendication 1, dans lequel :
ladite au moins une électrode (106) comprend une pluralité d'électrodes (106) ;
ledit au moins un paramètre de stimulation comprend une combinaison des électrodes (106) ;
le programmateur non implanté (122) est configuré pour provoquer un essai, par le neurostimulateur implantable (102), d'une pluralité de différentes combinaisons des électrodes (106) pour identifier la combinaison des électrodes (106) qui entraîne des contractions musculaires indésirables lorsqu'elle est utilisée pour stimuler sélectivement au moins une partie du ganglion rachidien à une première fréquence ; et
le programmateur non implanté (122) est configuré pour augmenter la première fréquence jusqu'à une deuxième fréquence pour passer des contractions musculaires indésirables à la sensation de massage qui traite la douleur, après que la combinaison des électrodes (106) qui produit les contractions musculaires indésirables a été identifiée.

8. Système (100) selon la revendication 7, dans lequel :
la première fréquence est inférieure ou égale à environ 10 Hz ; et
la deuxième fréquence est d'au moins 16 Hz, et/ou
le système comprend en outre un capteur configuré pour détecter les contractions musculaires indésirables.

9. Système selon la revendication 7, le système comprenant :
un moyen pour provoquer un essai d'une pluralité de différents paramètres de stimulation qui sont utilisés pour une stimulation sélective d'au moins une partie d'un ganglion rachidien associé à la douleur chez un patient à traiter ;
un moyen pour identifier le moment où la stimulation sélective produit une sensation de massage pour traiter la douleur ; et
un moyen pour sauvegarder des informations correspondant à au moins un paramètre de stimulation qui produit la sensation de massage qui traite la douleur.

10. Système selon la revendication 9, dans lequel le moyen d'identification comprend un moyen pour recevoir une indication selon laquelle la stimulation sélective produit une sensation de massage pour le traitement de la douleur.

11. Système selon la revendication 9, dans lequel le moyen d'identification comprend un moyen pour détecter le moment où la stimulation sélective, ayant une fréquence inférieure ou égale à 10 Hz, produit des contractions musculaires indésirables indiquant qu'au moins une électrode utilisée pour produire la stimulation sélective est positionnée près du ganglion rachidien associé à la douleur, préférablement
dans lequel le moyen d'identification comprend en outre un moyen pour augmenter la fréquence jusqu'à au moins 16 Hz pour passer des contractions musculaires indésirables à la sensation de massage pour le traitement de la douleur.

12. Support lisible par ordinateur, contenant des instructions stockées sur ledit support qui, lorsqu'elles sont lues et exécutées par un ou plusieurs processeurs, entraînent l'exécution par lesdits un ou plusieurs processeurs des étapes comprenant :
un essai d'une pluralité de différents paramètres de stimulation qui sont utilisés pour une stimulation sélective d'au moins une partie d'un ganglion rachidien associé à la douleur chez un patient à traiter ;
l'identification du moment où la stimulation sélective produit une sensation de massage pour traiter la douleur ; et
l'enregistrement d'informations correspondant à au moins un paramètre de stimulation qui produit la sensation de massage pour le traitement de la douleur, **caractérisé en ce que** les étapes comprennent en outre l'ajustement d'une fréquence de stimulation, d'une fréquence supérieure à 10 Hz à une fréquence à laquelle une indication est d'abord reçue d'un capteur, selon laquelle une contraction musculaire est détectée, avec ensuite une augmentation de la fréquence de stimulation jusqu'à une fréquence de 16 à 20 Hz pour passer au traitement.

13. Support lisible par ordinateur selon la revendication 12, dans lequel l'identification comprend la réception d'une indication selon laquelle la stimulation sélective produit une sensation de massage qui traite la douleur.

14. Support lisible par ordinateur selon la revendication 12, comprenant en outre l'identification du moment où la stimulation sélective produit une contraction musculaire indésirable indiquant qu'au moins une électrode utilisée pour produire la stimulation sélective est positionnée près du ganglion rachidien associé à la douleur, préférablement
dans lequel l'identification du moment où la stimulation sélective produit une contraction musculaire indésirable comprend la détection du moment où la stimulation sélective produit une contraction musculaire indésirable.

15. Support lisible par ordinateur selon la revendication 14, dans lequel l'identification comprend en outre l'augmentation d'une fréquence inférieure ou égale à 10 Hz jusqu'à une fréquence d'au moins 16 Hz pour passer de la contraction musculaire indésirable à la sensation de massage pour le traitement de la douleur.
